# EUROPEAN PATENT APPLICATION

(11) **EP 4 428 884 A1**
(43) Date of publication of application: **11.09.2024**
(21) Application number: 22889860.7
(22) Date of filing: 26.10.2022
(51) Int. Cl.: H01G 9/028, C07D 495/04, C08G 61/12, C08L 65/00, H01B 1/12, H01G 9/00, H10K 50/00

(54) **ELECTRIC OR ELECTRONIC DEVICE, METHOD FOR PRODUCING ELECTRIC OR ELECTRONIC DEVICE, AND APPARATUS COMPRISING SAID ELECTRIC OR ELECTRONIC DEVICE**

(30) Priority: 02.11.2021 JP 2021179842; 01.06.2022 JP 2022089542
(71) Applicant: The University of Tokyo, Bunkyo-ku, Tokyo 113-8654 (JP); Daicel Corporation, Osaka-shi, Osaka 530-0011 (JP)
(72) Inventor: SATO, Hiroki, Tokyo 108-8230 (JP); YAMASHITA, Yu, Tokyo 113-8654 (JP); TAKEYA, Junichi, Tokyo 113-8654 (JP); WATANABE, Shunichiro, Tokyo 113-8654 (JP)
(74) Representative: Grünecker Patent- und Rechtsanwälte PartG mbB
(86) International application number: PCT/JP2022/040018
(87) International publication number: WO 2023/080040

(57) **Abstract**

To provide an electric or electronic device having excellent heat resistance and moisture resistance. The electric or electronic device of the present disclosure having a configuration including a first electrode layer, a conductive material layer, and a second electrode layer that are layered in this order,
the conductive material layer containing a conductive material having a configuration in which a conjugated macromolecular compound is doped with an anion selected from the group consisting of a nitrogen anion, a boron anion, a phosphorus anion, and an antimony anion. The anion is preferably an anion represented by Formula (a-1) or (a-2).

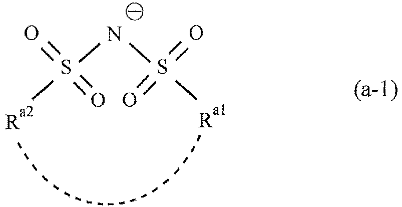

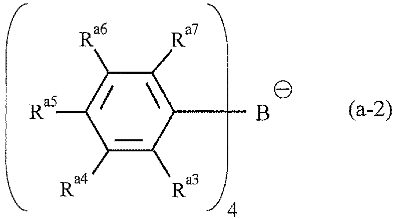

## Description

### Technical Field

The present disclosure relates to an electric or electronic device, a method for manufacturing the electric or electronic device, and apparatus including the electric or electronic device. The present disclosure claims priority to JP 2021-179842 filed in Japan on November 2, 2021 and JP 2022-089542 filed in Japan on June 1, 2022, the contents of which are incorporated herein.

### Background Art

As the electric or electronic device, for example, use of a solid-state electrolytic capacitor has been increasing, and examples of such use include use for a mobile device such as a cellular phone, a notebook computer, or a handheld game console, and use for a device for an automobile such as car navigation equipment and an engine control circuit.

The solid-state electrolytic capacitor includes a positive electrode layer, a dielectric layer, a solid-state electrolyte layer, and a negative electrode layer. It is known that a solid-state electrolytic capacitor including a solid-state electrolyte layer containing an electrically conductive macromolecular compound has excellent electrical characteristics and reduces an equivalent series resistance (ESR) value.

As the electrically conductive macromolecular compound, a polyethylenedioxythiophene doped with a polystyrene sulfonic acid (hereinafter, may be also referred to as "PEDOT/PSS") has been known (e.g., Patent Document 1).

### Citation List

### Patent Document

Patent Document 1: WO 2007/091656

### Summary of Invention

### Technical Problem

However, because a PEDOT/PSS has a low heat resistance and an electric conductivity remarkably decreases due to de-doping, decomposition of main chain backbone, or the like in a high temperature environment at higher than 100°C, it has been difficult for the PEDOT/PSS to be used for a purpose requiring the PEDOT/PSS to be exposed to a high temperature, such as an engine control circuit of an automobile.

Furthermore, a PSS has a high hygroscopicity. In forming a solid-state electrolyte layer containing a PEDOT/PSS, water is typically used. Thus, a solid-state electrolyte layer containing a PEDOT/PSS readily contains moisture in essence and tends to generate sulfonic acid, which is a strong acid. Therefore, when a solid-state electrolyte layer containing a PEDOT/PSS and a dielectric layer are layered, the dielectric layer is corroded due to the sulfonic acid generated from the solid-state electrolyte layer, the ESR value readily increases, and stable maintenance of electrical characteristics is difficult.

That is, in an electric or electronic device including a solid-state electrolyte layer containing a PEDOT/PSS, moisture resistance has been poor and deterioration of electrical characteristics has been remarkable due to moisture.

An object of the present disclosure is to provide an electric or electronic device having excellent heat resistance and moisture resistance.

Another object of the present disclosure is to provide a method for manufacturing the electric or electronic device.

Another object of the present disclosure is to provide an electric or electronic apparatus including the electric or electronic device.

### Solution to Problem

As a result of diligent research to solve the issues described above, the inventors of the present invention found the following:
in a conductive material produced by doping a conjugated macromolecular compound with an anion selected from the group consisting of a nitrogen anion, a boron anion, a phosphorus anion, and an antimony anion, the anion is stably contained in a gap of a crystalline structure of the conjugated macromolecular compound, and thus de-doping is less likely to occur even in a high temperature environment;
the conductive material has a lower hygroscopicity compared to a PEDOT/PSS and can reduce an amount of water used in a manufacturing process, and by reducing the amount of water, it is possible to suppress a water content essentially contained in the conductive material; and
an electric or electronic device having a layered structure of an electrode layer and a conductive material layer containing the conductive material as a main component can suppress de-doping even in a high temperature environment, can maintain electrical conductivity, can suppress moisture absorption of the conductive material layer even in a high humidity environment, and thus can suppress corrosion of an easily corrodible layer, such as an electrode layer and a dielectric layer, caused by a moisture content of the conductive material layer. The present disclosure has been completed based on these findings.

That is, the present disclosure provides an electric or electronic device having a configuration including a first electrode layer, a conductive material layer, and a second electrode layer that are layered in this order,
the conductive material layer containing a conductive material having a configuration in which a conjugated macromolecular compound is doped with an anion selected from the group consisting of a nitrogen anion, a boron anion, a phosphorus anion, and an antimony anion.

The present disclosure also provides the electric or electronic device, where the anion is an anion represented by Formula (a-1) or (a-2): 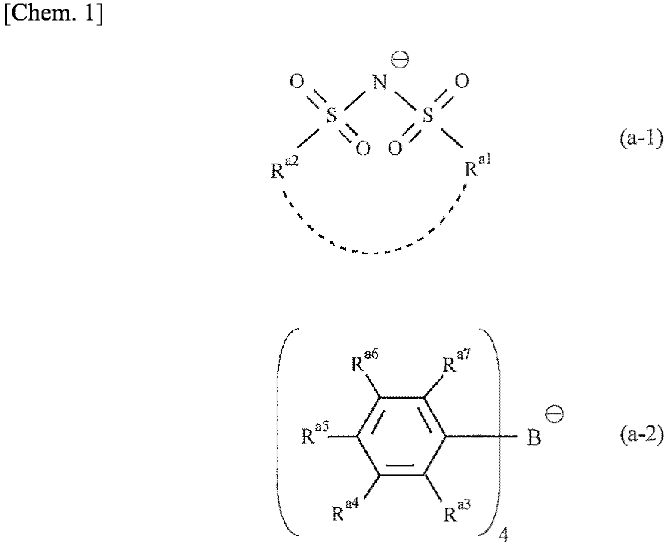 where R^{a1} to R^{a7} are identical or different and each represent a halogen atom or a haloalkyl group; and R^{a1} and R^{a2} may be bonded to each other to form a haloalkylene group.

The present disclosure also provides the electric or electronic device, in which the conjugated macromolecular compound is a macromolecular compound containing at least one selected from the group consisting of repeating units represented by Formulas (1a) to (1c) or a macromolecular compound containing a repeating unit represented by Formula (2): 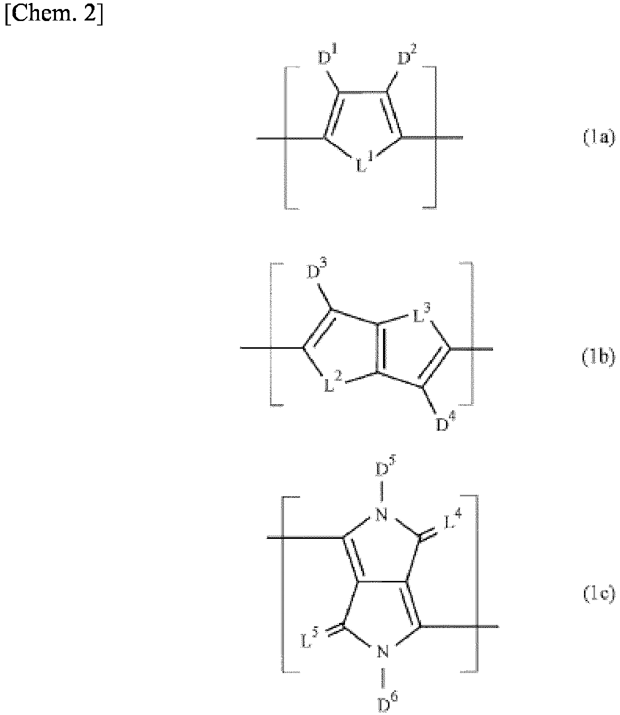 where L¹ to L⁵ are identical or different and each represent an element of Groups 13 to 16; and D¹ to D⁶ are identical or different and each represent a group selected from the group consisting of an alkyl group, a haloalkyl group, an electron donating group containing a heteroatom, and a hydrogen atom, with proviso that at least one selected from the group consisting of D¹ to D⁶ is a group selected from the group consisting of an alkyl group, a haloalkyl group, and an electron donating group containing a heteroatom; 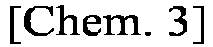 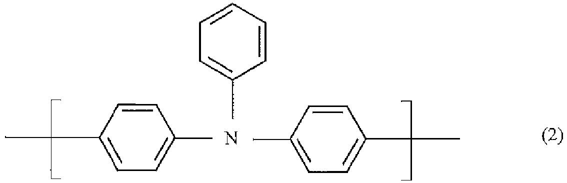 where a benzene ring may contain a substituent.

The present disclosure also provides the electric or electronic device, in which the macromolecular compound containing at least one selected from the group consisting of repeating units represented by Formulas (1a) to (1c) is a macromolecular compound containing a repeating unit represented by Formula (1-1): 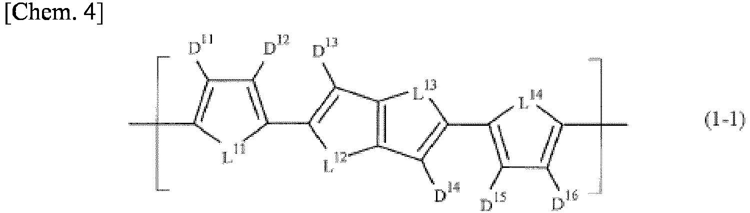 where L¹¹ to L¹⁴ are identical or different and each represent an element of Groups 13 to 16; and D¹¹ to D¹⁶ are identical or different and each represent a group selected from the group consisting of an alkyl group, a haloalkyl group, an electron donating group containing a heteroatom, and a hydrogen atom, with proviso that at least one selected from the group consisting of D¹¹ to D¹⁶ is a group selected from the group consisting of an alkyl group, a haloalkyl group, and an electron donating group containing a heteroatom.

The present disclosure also provides the electric or electronic device, in which at least one of D¹¹ to D¹⁶ in Formula (1-1) is a group represented by Formula (d-1) or (d-2): 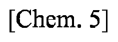 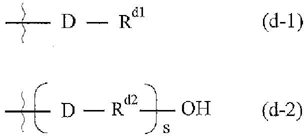 where D represents at least one heteroatom selected from the group consisting of elements of Groups 14 to 16; R^{d1} represents an aliphatic hydrocarbon group having from 5 to 30 carbons; R^{d2} represents an aliphatic hydrocarbon group having from 1 to 5 carbons, and s represents an integer of 1 or greater; and a bond indicated by a wavy line in the formulas is bonded to a main chain of the macromolecular compound containing a repeating unit represented by Formula (1-1).

The present disclosure also provides the electric or electronic device, in which the electric or electronic device is an electrolytic capacitor, an organic electroluminescent element, or an organic solar cell.

The present disclosure also provides a method for manufacturing an electric or electronic device described above, the method including:
doping a conjugated macromolecular compound with a dopant.

The present disclosure also provides a method for manufacturing an electric or electronic device described above, the method including:
1: forming a layer containing a conjugated macromolecular compound by applying a solution containing the conjugated macromolecular compound to a substrate surface and drying the solution on the substrate surface; and
2: producing a conductive material layer containing a conductive material by applying a dopant solution to the layer containing the conjugated macromolecular compound and drying the dopant solution on the layer containing the conjugated macromolecular compound, the dopant solution containing a salt containing an anion and a counter cation, the anion being selected from the group consisting of a nitrogen anion, a boron anion, a phosphorus anion, and an antimony anion, and the conductive material having a configuration in which the conjugated macromolecular compound is doped with the anion.

The present disclosure also provides the method for manufacturing an electric or electronic device, in which a moisture content of the dopant solution is 1 wt.% or less.

The present disclosure also provides an electric or electronic apparatus including the electric or electronic device described above.

### Advantageous Effects of Invention

The electric or electronic device of the present disclosure includes a conductive material layer having excellent heat resistance and being capable of suppressing de-doping even in a high temperature environment. The conductive material layer also has excellent moisture resistance. That is, the conductive material layer essentially has a low moisture content and, in addition, has a low hygroscopicity. Since the electric or electronic device includes a conductive material layer having excellent moisture resistance as described above, corrosion of an easily corrodible layer, such as an electrode layer or a dielectric layer, can be prevented even in a high humidity environment, and high electrical characteristics can be maintained.

The electric or electronic device of the present disclosure can stably exhibit excellent electrical characteristics for a long period of time even in a high temperature and high humidity environment. Thus, the electric or electronic device can be suitably used for a purpose requiring moisture resistance and heat resistance (e.g., devices for automobiles). In addition, according to the electric or electronic device, weight reduction, flexibility, thickness reduction, and area increase can be realized at low cost.

### Description of Embodiments

### Electric or Electronic Device

The electric or electronic device of the present disclosure (hereinafter, simply referred to as "device") has a configuration including a first electrode layer, a conductive material layer, and a second electrode layer that are layered in this order. The conductive material layer contains a conductive material having a configuration in which a conjugated macromolecular compound is doped with an anion selected from the group consisting of a nitrogen anion, a boron anion, a phosphorus anion, and an antimony anion.

The device may include another configuration as necessary, in addition to the first electrode layer, the conductive material layer, and the second electrode layer.

Examples of the device include an electrolytic capacitor (e.g., solid-state electrolytic capacitor), an organic electroluminescent element (hereinafter, may be referred to as "organic EL element"), and an organic solar cell (e.g., organic thin-film solar cell).

In a case where the device is an electrolytic capacitor, a layered body including a positive electrode layer as a first electrode layer, a conductive material layer (this is also referred to as an electrolyte layer), and a negative electrode layer as a second electrode layer is provided. The layered body preferably includes a dielectric layer between the positive electrode layer and the conductive material layer. Furthermore, a graphite layer may be included between the negative electrode layer and the conductive material layer. By providing the graphite layer, connection between the conductive material layer and the second electrode layer can be improved.

In a case where the device is an organic EL element, a layered body including a metal electrode as a first electrode layer, a conductive material layer (this is also referred to as a hole injection layer), and a transparent electrode (anode) as a second electrode layer is provided. The layered body preferably include an emission layer between the metal electrode and the conductive material layer and preferably includes a transparent substrate on an outer side of the transparent electrode (anode).

In a case where the device is an organic solar cell, a layered body including a positive electrode layer as a first electrode layer, a conductive material layer (this is also referred to as an acceptor layer), and a negative electrode layer as a second electrode layer is provided. The layered body preferably includes a donor layer between the positive electrode layer and the conductive material.

Hereinafter, a solid-state electrolytic capacitor will be mainly described; however, the electric or electronic device of the present disclosure is not limited to the solid-state electrolytic capacitor.

In a case where the device is a solid-state electrolytic capacitor, the device has low ESR properties, and an ESR value measured at a frequency of 100 kHz using an LCR meter is, for example, 3.5 mΩ/cm² or less, preferably 3.0 mΩ/cm² or less, particularly preferably 2.0 mΩ/cm² or less, and most preferably 1.8 mΩ/cm² or less.

An ESR value measured at a frequency of 120 kHz using an LCR meter is, for example, 1.0 mΩ/cm² or less, preferably 0.5 mΩ/cm² or less, and particularly preferably 0.4 mΩ/cm² or less.

The solid-state electrolytic capacitor has excellent heat resistance, and a percentage of ESR increase (a value determined by the following equation) after a heat resistance test in which heating is performed at 150°C for 2000 hours with respect to an ESR value before the test (25°C) is, for example, 45% or less, preferably 30% or less, and particularly preferably 25% or less. Percentage of ESR increase = [(ESR value after heat resistance test - ESR value before test)/ESR value before test] × 100

Furthermore, the solid-state electrolytic capacitor has excellent moisture resistance and a percentage of increase of an ESR value after a moisture resistance test, in which the solid-state electrolytic capacitor is allowed to stand under conditions of 25°C and 85%RH for 3 days, from an ESR value before the test (25°C, 50%RH) (a value determined by the following equation) is, for example, 50% or less, preferably 40% or less, more preferably 30% or less, particularly preferably 15% or less, most preferably 10% or less, and especially preferably 6% or less. Percentage of ESR increase = [(ESR value after moisture resistance test - ESR value before test)/ESR value before test] × 100

### Conductive Material Layer

The conductive material layer contains a conductive material (that is, electrically conductive macromolecular compound). The conductive material layer may contain a component other than the conductive material; however, the proportion of the conductive material is, for example, 50 wt.% or greater, preferably 60 wt.% or greater, more preferably 70 wt.% or greater, particularly preferably 80 wt.% or greater, most preferably 90 wt.% or greater, and especially preferably 95 wt.% or greater, with respect to the total amount of the conductive material layer. Note that the upper limit of the proportion is 100 wt.%.

The conductive material layer at least contains a conductive material having a configuration in which a conjugated macromolecular compound is doped with an anion. The conductive material layer may contain another conductive material in addition to the conductive material described above; however, the proportion of such another conductive material is, for example, 50 wt.% or less, preferably 40 wt.% or less, more preferably 30 wt.% or less, particularly preferably 20 wt.% or less, most preferably 10 wt.% or less, and especially preferably 5 wt.% or less, with respect to the total amount of the conductive material layer.

The electric conductivity of the conductive material layer (or the conductive material) is, for example, 1 S/cm or greater, preferably 10 S/cm or greater, particularly preferably 50 S/cm or greater, most preferably 100 S/cm or greater, and especially preferably 500 S/cm or greater. The upper limit of the electric conductivity is, for example, 2000 S/cm.

The conductive material layer (or the conductive material) has excellent storage stability and can maintain high electrical conductivity over a long period of time. For example, a percentage of decrease in electric conductivity after storage at room temperature (25°C) for 14 days ([(electric conductivity before storage - electric conductivity after storage for 14 days)/electric conductivity before storage] × 100) is 5% or less, preferably 3% or less, and particularly preferably 1% or less.

A percentage of decrease in electric conductivity after storage at room temperature (25°C) for 56 days ([(electric conductivity before storage - electric conductivity after storage for 56 days)/electric conductivity before storage] × 100) is 20% or less, preferably 15% or less, and particularly preferably 10% or less.

Furthermore, the conductive material layer (or the conductive material) has a low hygroscopicity, and the moisture absorption amount when a moisture resistance test, in which the conductive material layer (or the conductive material) is allowed to stand under conditions of 25°C and 85%RH for 3 days, is performed is, for example, 1 wt.% or less, preferably 0.5 wt.% or less, and particularly preferably 0.1 wt.% or less.

### Conjugated Macromolecular Compound

The conjugated macromolecular compound is a macromolecular compound having a suitability for doping and has an ionization potential of preferably 6.0 eV or less, more preferably 5.5 eV or less, particularly preferably 5.0 eV or less, and most preferably 4.5 eV or less. Note that the ionization potential can be determined by the photoemission yield spectroscopy.

The conjugated macromolecular compound is preferably a macromolecular compound having a low hygroscopicity and excellent solubility in an organic solvent (e.g., aromatic hydrocarbon such as benzene, toluene, and xylene).

The weight average molecular weight of the conjugated macromolecular compound is, for example, from 500 to 300000.

The conjugated macromolecular compound includes a macromolecular compound (1) and a macromolecular compound (2) described below. Note that the macromolecular compounds (1) and (2) also include oligomers.

Because the macromolecular compounds (1) and (2) have low hygroscopicity, a conductive material layer (or conductive material) produced by using these as the conjugated macromolecular compound can also suppress moisture absorption even in a high humidity environment. Thus, the solid-state electrolytic capacitor including the conductive material layer achieves an excellent effect of suppressing corrosion of the dielectric layer even in a high humidity environment, can suppress increase in a leakage current, and can stably maintain low ESR characteristics for a long period of time.

### Macromolecular Compound (1)

The macromolecular compound (1) contains at least one selected from the group consisting of repeating units represented by Formulas (1a), (1b), and (1c) below.

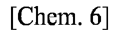

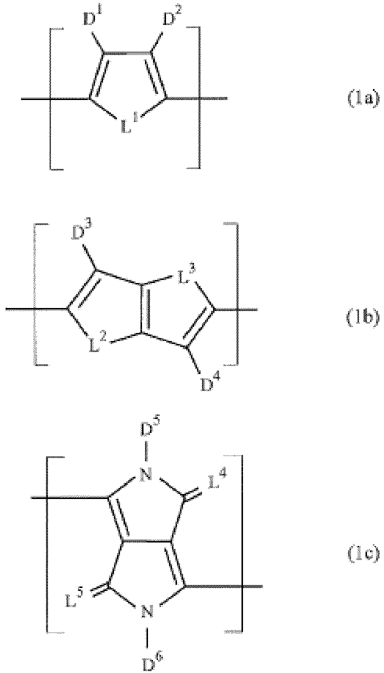

In the formulas, L¹ to L⁵ are identical or different and each represent an element of Groups 13 to 16; and D¹ to D⁶ (these may be referred to as side chains) are identical or different and each represent a group selected from the group consisting of an alkyl group, a haloalkyl group, an electron donating group containing a heteroatom, and a hydrogen atom. Note that at least one selected from the group consisting of D¹ to D⁶ is a group selected from the group consisting of an alkyl group, a haloalkyl group, and an electron donating group containing a heteroatom.

From the viewpoint of producing a conductive material layer having particularly excellent heat resistance, the macromolecular compound (1) is preferably a macromolecular compound containing repeating units represented by Formulas (1a) and (1b) above, and at least one selected from the group consisting of D¹ to D⁴ in the formula is preferably a group selected from the group consisting of an alkyl group, a haloalkyl group, and an electron donating group containing a heteroatom.

From the viewpoint of producing a conductive material layer having particularly excellent heat resistance, the macromolecular compound (1) is particularly preferably a macromolecular compound containing a repeating unit represented by Formula (1-1) below.

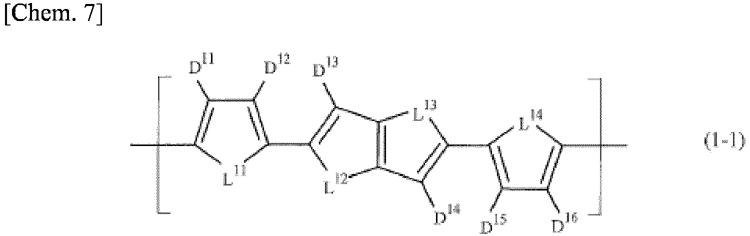

In the formula, L¹¹ to L¹⁴ are identical or different and each represent an element of Groups 13 to 16; and D¹¹ to D¹⁶ (these may be referred to as side chains) are identical or different and each represent a group selected from the group consisting of an alkyl group, a haloalkyl group, an electron donating group containing a heteroatom, and a hydrogen atom. Note that at least one selected from the group consisting of D¹¹ to D¹⁶ is a group selected from the group consisting of an alkyl group, a haloalkyl group, and an electron donating group containing a heteroatom.

As the macromolecular compound (1), a macromolecular compound containing a repeating unit represented by Formula (1-1-1) or (1-1-2)below is most preferred, and a macromolecular compound containing a repeating unit represented by Formula (1-1-2) below is especially preferred. In the formulas below, L¹¹ to L¹⁴ and D¹¹ to D¹⁶ are the same as those described above.

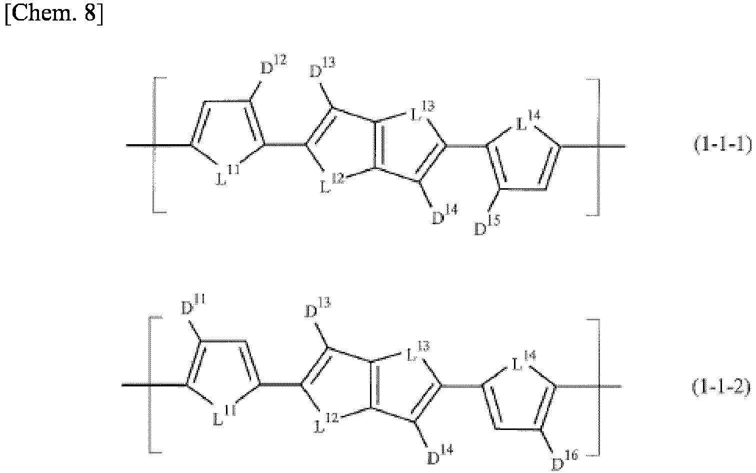

Examples of the elements of Groups 13 to 16 include boron atoms, nitrogen atoms, oxygen atoms, phosphorus atoms, and sulfur atoms. Among these, the element of Group 15 or 16 is preferred, and an oxygen atom or a sulfur atom is particularly preferred.

The alkyl group is preferably an alkyl group having from 3 to 30 carbons, particularly preferably an alkyl group having from 5 to 20 carbons, and most preferably an alkyl group having from 10 to 20 carbons. Furthermore, the alkyl group includes a straight-chain or branched alkyl group; however, a straight-chain alkyl group is particularly preferred.

The haloalkyl group is a group in which at least one hydrogen atom contained in an alkyl group is substituted with a halogen atom (e.g., fluorine atom or a chlorine atom), and examples of the alkyl group include those same as the alkyl group described above. Among these, the haloalkyl group is preferably an alkyl group having a halogen atom at a terminal, and particularly preferably an alkyl group having a fluorine atom at a terminal.

Examples of the electron donating group containing a heteroatom include a group containing at least one heteroatom selected from the elements of Groups 14 to 16.

Among these, the electron donating group containing a heteroatom is preferably a group represented by Formula (d-1) or (d-2) below. In the macromolecular compound (1) having a group represented by Formula (d-1) or (d-2) below in a side chain, the anion released from the ionic bond with the counter cation can be stably placed in a gap of the crystalline structure made of the side chain. Thus, de-doping can be suppressed, and a state with high conductivity can be maintained stably over time.

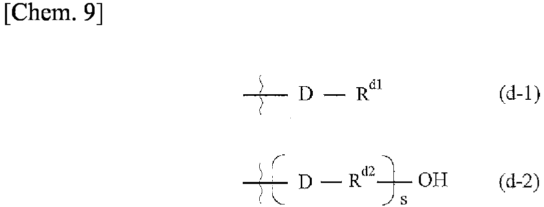

In the formula, D represents at least one heteroatom selected from the group consisting of elements of Groups 14 to 16; R^{d1} represents an aliphatic hydrocarbon group having from 5 to 30 carbons. R^{d2} represents an aliphatic hydrocarbon group having from 1 to 5 carbons, and s represents an integer of 1 or greater. A bond indicated by a wavy line in the formulas is bonded to a main chain of the macromolecular compound containing the repeating unit described above.

Examples of at least one heteroatom selected from the elements of Groups 14 to 16 include a nitrogen atom, an oxygen atom, a phosphorus atom, and a sulfur atom. Among them, the element of Group 15 or 16 is preferable, and an oxygen atom is particularly preferable.

The aliphatic hydrocarbon group in R^{d1} is a monovalent aliphatic hydrocarbon group having from 5 to 30 carbons, and examples thereof include alkyl groups having from 5 to 30 carbons (preferably from 5 to 20 carbons, particularly preferably from 10 to 20 carbons, most preferably from 10 to 18 carbons, and especially preferably from 12 to 15 carbons), such as a pentyl group, a hexyl group, a decyl group, a dodecyl group, a hexadecyl group, and a nonadecyl group; and alkenyl groups having from 5 to 30 carbons (preferably from 10 to 30 carbons, more preferably from 10 to 20 carbons, particularly preferably from 10 to 18 carbons, and most preferably from 12 to 18 carbons), such as an oleyl group.

Among these, the monovalent aliphatic hydrocarbon group in R^{d1} is preferably an alkyl group.

The aliphatic hydrocarbon group in R^{d2} is a divalent aliphatic hydrocarbon group having from 1 to 5 carbons, and examples thereof include a straight-chain or branched alkylene group, such as a methylene group, a methylmethylene group, a dimethylmethylene group, an ethylene group, a propylene group, and a trimethylene group.

Among these, the aliphatic hydrocarbon group in R^{d2} is preferably an alkylene group having from 2 to 4 carbons, and is particularly preferably an alkylene group having from 2 to 3 carbons.

s is the number of repetitions of the unit represented in the parentheses in Formula (d-2) and is an integer of 1 or greater. s is preferably an integer of 1 to 20, and particularly preferably an integer of 4 to 10.

From the viewpoint of an excellent de-doping suppression effect, the macromolecular compound (1) preferably contains an electron donating group containing a heteroatom (especially, a group represented by Formula (d-1)) as a side chain (at least one of D¹ to D⁶, at least one of D¹ to D⁴, or at least one of D¹¹ to D¹⁶) and preferably contains from 1 to 4 (preferably from 2 to 4, and particularly preferably from 2 to 3) of the electron donating group(s) containing a heteroatom in a repeating unit (preferably a repeating unit represented by Formula (1-1) above, and particularly preferably a repeating unit represented by Formula (1-1-1) or (1-1-2) above).

The macromolecular compound containing a repeating unit represented by Formula (1-1-1) above can be produced through reactions (I) and (II) below.

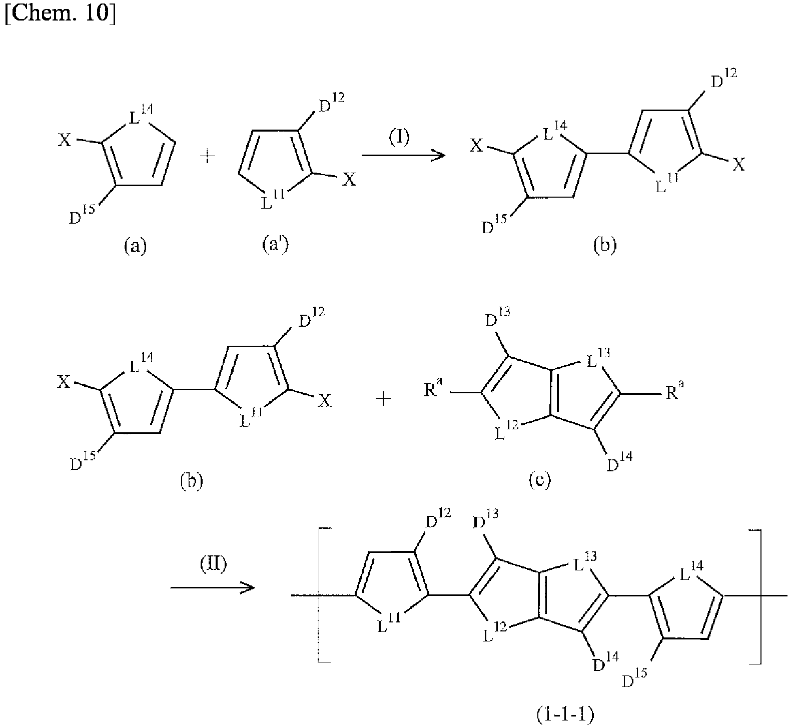

In the above formulas, D¹² to D¹⁵ and L¹¹ to L¹⁴ are the same as described above. X represents a halogen atom.

In the reaction (I), a compound represented by Formula (a) above and a compound represented by Formula (a') above are reacted to produce a compound represented by Formula (b) above.

The reaction (I) is preferably carried out in the presence of a silver catalyst and/or a base. Further, the reaction is preferably carried out in the presence of a palladium complex and/or a ligand.

Examples of the silver catalyst include silver salts such as silver oxide, silver carbonate, silver nitrate, silver sulfate, silver cyanide, silver chloride, silver bromide, silver iodide, silver acetate, silver benzoate, and silver lactate; and silver complexes such as silver acetylacetonate. One of these can be used alone or two or more in combination.

An amount of the silver catalyst used is, for example, approximately from 0.05 to 5.0 mol per mol of a total of the compound represented by Formula (a) above and the compound represented by Formula (a') above.

Examples of the base include organic bases such as triethylamine, tri-n-propylamine, tri-n-butylamine, tri-s-butylamine, tri-t-butylamine, diisopropylethylamine, dimethylcyclohexylamine, dicyclohexylethylamine, tribenzylamine, N-methylpiperidine, N,N-dimethylaniline, N,N-diethylaniline, 1,4-diazabicyclo[2.2.2]octane, tetramethylethylenediamine, 1,4-dimethylpiperazine, N-methylpyrrolidine, N-methylmorpholine, 1-methyl-2,2,6,6-tetramethylpiperidine, 1,5-diazabicyclo[4.3.0]-5-nonene, 1,8-diazabicyclo[5.4.0]-7-undecene, pyridine, 2,4-dimethylpyridine, 2,4,6-trimethylpyridine, 4-dimethylaminopyridine, and 2,6-di-t-butylpyridine; and alkali metal alkoxides such as sodium methoxide, sodium ethoxide, and potassium t-butoxide; alkali metal carbonates such as sodium hydrogen carbonate, sodium carbonate, potassium carbonate, rubidium carbonate, and cesium carbonate; alkali metal phosphates such as potassium phosphate; alkali metal hydroxides such as lithium hydroxide, sodium hydroxide, and potassium hydroxide; metal amides such as lithium diisopropylamide; and halides of alkali metals such as potassium fluoride, potassium iodide, sodium fluoride, and cesium fluoride. One of these can be used alone or two or more in combination.

An amount of the base used is approximately from 0.5 to 5.0 mol per mol of a total of the compound represented by Formula (a) and the compound represented by Formula (a').

Examples of the palladium catalyst include zero-valent palladium compounds such as tetrakistriphenylphosphine palladium, bis(1,5-cyclooctadiene)palladium, bis(triphenylphosphine)(maleic anhydride)palladium, tris(dibenzylideneacetone)dipalladium, and (1,5-cyclooctadiene)(maleic anhydride)palladium; and divalent palladium compounds such as palladium acetate, palladium propionate, palladium carbonate, palladium benzoate, palladium acetylacetonate, palladium chloride, palladium sulfate, palladium nitrate, lithium palladium chloride, bisbenzonitrile palladium chloride, bistriphenylphosphine palladium chloride, bistriphenylphosphine palladium acetate, π-allylpalladium chloride, π-allylpalladium acetate, and sodium tetrachloroparadate. One of these can be used alone or two or more in combination.

An amount of the palladium catalyst used is, for example, approximately from 1 to 10 mol% of the compound represented by Formula (a) above and the compound represented by Formula (a') above.

The reaction (I) can be performed in the presence of a solvent. Examples of the solvent include nitrile-based solvents such as acetonitrile, propionitrile, and benzonitrile; water; alcohol-based solvents such as methanol; amide-based solvents such as N,N-dimethylformamide and N,N-dimethylacetamide; ether-based solvents such as diethyl ether, THF, and dioxane; ester-based solvents such as ethyl acetate; dimethylformamide and dimethylsulfoxide. One of these can be used alone or two or more in combination.

A reaction atmosphere in the reaction (I) is any atmosphere that does not inhibit the reaction and not particularly limited, and may be, for example, any of an air atmosphere, a nitrogen atmosphere, and an argon atmosphere.

A reaction temperature of the reaction (I) is, for example, approximately from 30 to 100°C. A reaction time is, for example, approximately from 0.5 to 5 hours.

The reaction (II) is a cross-coupling reaction between the compound represented by Formula (b) above and a compound represented by Formula (c) above. As the cross-coupling reaction, for example, a Stille cross-coupling reaction, a Kumada cross-coupling reaction, a Negishi cross-coupling reaction, a Suzuki cross-coupling reaction, or the like can be used. Through this reaction, the macromolecular compound having the repeating unit represented by Formula (1-1-1) above is produced.

The compound represented by Formula (c) above can be selected and used depending on the type of cross-coupling reaction used.

For example, when the Stille cross-coupling reaction is utilized, the compound represented by Formula (c) above is an organotin compound, and R^{a} in Formula (c) above is a trialkylstannyl group (SnR₃ group, where R is, for example, an alkyl group having from 1 to 3 carbons).

When the Kumada cross-coupling reaction is utilized, the compound represented by Formula (c) above is a magnesium compound, and R^{a} in Formula (c) is a magnesium halide group (-MgX group, where X represents a halogen atom).

When the Negishi cross-coupling reaction is utilized, the compound represented by Formula (c) above is a zinc compound, and R^{a} in Formula (c) above is a ZnX group (where X represents a halogen atom).

When the Suzuki cross-coupling reaction is utilized, the compound represented by Formula (c) above is a boron compound, and R^{a} in Formula (c) above is a BY₂ group (where Y is, for example, a hydroxyl group).

An amount of the used compound represented by Formula (c) above is, for example, approximately from 1.0 to 1.5 mol per mol of the compound represented by Formula (b) above.

The reaction (II) is preferably carried out in the presence of a palladium catalyst and/or a ligand.

Examples of the palladium catalyst include Pd(PPh₃)₄, Pd(dba)₂, Pd₂(dba)₃, Pd₂(dba)₃·CHCl₃, Pd(t-Bu₃P)₂, and Pd(acac)₂. One of these can be used alone or two or more in combination.

The amount of the palladium catalyst used is, for example, approximately from 0.5 to 10 mol% of the compound represented by Formula (b) above.

The ligand is preferably a phosphine ligand. Examples of the phosphine ligand include tri(C₁₋₅)alkyl phosphines such as trimethylphosphine and tri(t-butyl)phosphine; and tri(C₃₋₆)cycloalkylphosphines such as tri(cyclohexyl)phosphine; and aromatic phosphines such as tri(o-tolyl)phosphine, dimethylphenylphosphine, and diphenyl-2-pyridinylphosphine. One of these can be used alone or two or more in combination.

The amount of the ligand used is, for example, approximately from 1 to 15 mol% of the compound represented by Formula (b) above.

The reaction (II) can be performed in the presence of a solvent. Examples of the solvent include aromatic hydrocarbons such as benzene, toluene, xylene, ethylbenzene, trifluorotoluene, chlorobenzene, anisole, benzonitrile, nitrobenzene, and ethyl benzoate; aliphatic hydrocarbons such as hexane, heptane, and octane; alicyclic hydrocarbons such as cyclohexane; haloalkanes such as carbon tetrachloride, chloroform, dichloromethane, and 1,2-dichloroethane; ketones such as acetone and methyl ethyl ketone; esters such as methyl acetate, ethyl acetate, isopropyl acetate, and butyl acetate; amides such as N,N-dimethylformamide and N,N-dimethylacetamide; nitriles such as acetonitrile and propionitrile; chain or cyclic ethers such as diethyl ether, dibutyl ether, dimethoxyethane, dioxane, and tetrahydrofuran; and organic acids such as acetic acid. One of these can be used alone or two or more in combination.

A reaction atmosphere in the reaction (II) is any atmosphere that does not inhibit the reaction and not particularly limited, and may be, for example, any of an air atmosphere, a nitrogen atmosphere, and an argon atmosphere.

A reaction temperature of the reaction (II) is, for example, approximately from room temperature (25°C) to 200°C. A reaction time is, for example, approximately from 1 to 72 hours.

The macromolecular compound containing a repeating unit represented by Formula (1-1-2) above can be produced through reactions (III) and (IV) below.

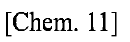

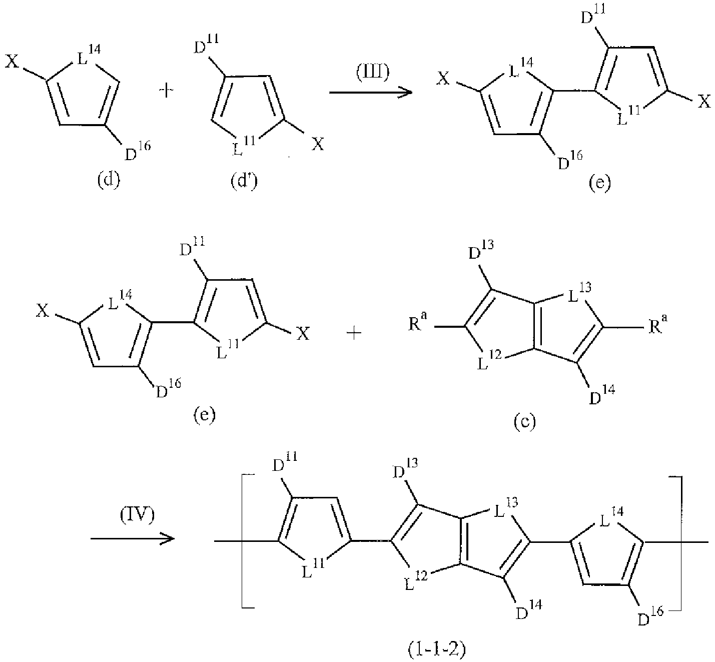

In the above formulas, D¹¹, D¹³, D¹⁴, D¹⁶, L¹¹ to L¹⁴, X, and R^{a} are the same as described above.

In the reaction (III), a compound represented by Formula (d) above and a compound represented by Formula (d') above are reacted to produce a compound represented by Formula (e) above.

The reaction (III) is preferably carried out in the presence of a copper catalyst and/or a base.

Examples of the copper catalyst include monovalent or divalent copper halides such as copper (I) iodide, copper (II) iodide, copper (I) bromide, copper (II) bromide, copper (I) chloride, copper (II) chloride, copper (I) fluoride, and copper (II) fluoride; and copper salts of monovalent or divalent protonic acids such as copper (I) oxide, copper (II) oxide, copper (I) sulfate, copper (II) sulfate, copper (I) carbonate, copper (II) carbonate, copper (I) acetate, copper (II) acetate, copper (I) nitrate, copper (II) nitrate, copper (I) methanesulfonate, copper (II) methanesulfonate, copper (I) trifluoromethanesulfonate, copper (II) trifluoromethanesulfonate, copper (I) cyanide, and copper (II) cyanide. One of these can be used alone or two or more in combination.

An amount of the copper catalyst used is, for example, approximately from 0.05 to 1 mol per mol of a total of the compound represented by Formula (d) above and the compound represented by Formula (d') above.

Examples of the base include the same examples as those which can be used in the reaction (I). One of the bases can be used alone or two or more in combination.

An amount of the base used is approximately from 0.5 to 5.0 mol per mol of a total of the compound represented by Formula (d) above and the compound represented by Formula (d') above.

The reaction (III) can be performed in the presence of a solvent. Examples of the solvent include the same examples as those which can be used in reaction (I). One of the solvents can be used alone, or two or more in combination.

A reaction atmosphere in the reaction (III) is any atmosphere that does not inhibit the reaction and not particularly limited, and may be, for example, any of an air atmosphere, a nitrogen atmosphere, and an argon atmosphere.

A reaction temperature of the reaction (III) is, for example, approximately from -100 to 10°C. A reaction time is, for example, approximately from 0.5 to 5 hours.

The reaction (IV) is a cross-coupling reaction between the compound represented by Formula (e) above and a compound represented by Formula (c) above. The reaction (IV) can be carried out in a similar manner as in the reaction (II), except that the compound represented by Formula (e) above is used instead of the compound represented by Formula (b) above.

### Macromolecular Compound (2)

The macromolecular compound (2) contains a repeating unit represented by Formula (2) below. The benzene ring in Formula (2) below may contain one or two or more substituents. Examples of the substituent include a halogen atom and a hydrocarbon group having from 1 to 5 carbons (e.g., methyl group).

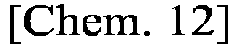

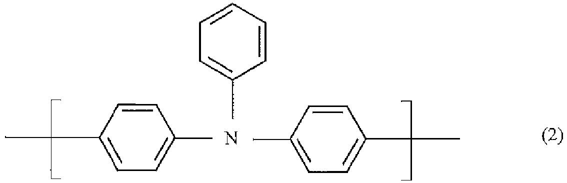

### Anion

The anion (or dopant anion) is at least one selected from the group consisting of a nitrogen anion, a boron anion, a phosphorus anion, and an antimony anion.

Among these, the anion is preferably a nitrogen anion or a boron anion from the viewpoint of an excellent corrosion suppression effect for an easily corrodible layer, such as an electrode layer or a dielectric layer. Furthermore, after being released from the ionic bond with the counter cation, the nitrogen anion and the boron anion are stably placed in the gap of the crystalline structure of the conjugated macromolecular compound, crystallinity of the conjugated macromolecular compound is increased, and thus the nitrogen anion and the boron anion are also preferred from the viewpoint of an excellent de-doping suppression effect.

The nitrogen anion is, for example, represented by Formula (a) below.

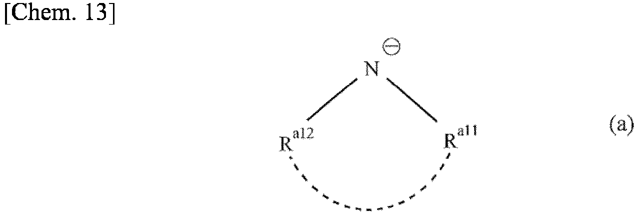

In the formula, R^{a11} and R^{a12} are identical or different and each represent an electron withdrawing group. R^{a11} and R^{a12} may be bonded to each other to form a ring with an adjacent nitrogen atom.

Examples of the electron-withdrawing group include a nitro group, a cyano group, a (C₁₋₅) acyl group, a carboxyl group, a (C₁₋₅) alkoxycarbonyl group, a halo (C₁₋₅) alkyl group, a sulfo group, a (C₁₋₅) alkylsulfonyl group, a halosulfonyl group, and a halo (C₁₋₅) alkylsulfonyl group.

Among these, R^{a11} and R^{a12} are each preferably a halosulfonyl group or a haloalkylsulfonyl group. Furthermore, R^{a11} and R^{a12} may be bonded to each other to form a sulfonyl-haloalkylene-sulfonyl group.

Examples of the halosulfonyl group include a fluorosulfonyl group and a chlorosulfonyl group.

Examples of the haloalkylsulfonyl group include halo(Ci-s)alkylsulfonyl groups, such as fluoroalkylsulfonyl groups (e.g., fluoro(C₁₋₅)alkylsulfonyl groups, such as a fluoromethylsulfonyl group, a trifluoroethylsulfonyl group, a trifluoropropylsulfonyl group, and a pentafluoropropylsulfonyl group; and perfluoro(C₁₋₅)alkylsulfonyl groups, such as a trifluoromethylsulfonyl group, a pentafluoroethylsulfonyl group, a pentafluoropropylsulfonyl group, and a nonafluorobutylsulfonyl group); and chloroalkylsulfonyl groups (e.g., chloro(C₁₋₅)alkylsulfonyl groups, such as a chloromethylsulfonyl group).

Examples of the haloalkylene group in the sulfonyl-haloalkylene-sulfonyl group formed by bonding R^{a11} and R^{a12} to each other include halo(C₁₋₅)alkylene group, such as fluoroalkylene groups (e.g., perfluoro(C₁₋₅)alkylene groups, such as a tetrafluoroethylene group, a hexafluoropropane-1,3-diyl group, and an octafluorobutane-1,4-diyl group), and chloroalkylene groups (e.g., perchloro(C₁₋₅)alkylene groups).

The nitrogen anion is particularly preferably an anion represented by Formula (a-1) below from the viewpoint of low hygroscopicity. When the anion is used, a conductive material having a particularly low hygroscopicity can be formed, and corrosion of an easily corrodible layer, such as an electrode layer or a dielectric layer, caused by moisture content of the conductive material can be remarkably suppressed.

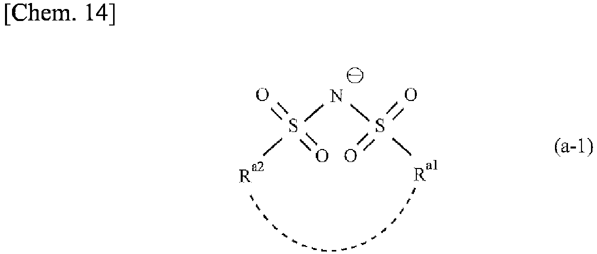

In the formula, R^{a1} and R^{a2} are identical or different and each represent a halogen atom or a haloalkyl group. R^{a1} and R^{a2} may be bonded to each other to form a haloalkylene group.

Examples of the haloalkyl group include halo(C₁₋₅)alkyl groups, such as fluoroalkyl groups (e.g., fluoro(C₁₋₅)alkyl groups, such as a fluoromethyl group, a trifluoroethyl group, a trifluoropropyl group, and a pentafluoropropyl group; and perfluoro(C₁₋₅)alkyl groups, such as a trifluoromethyl group, a pentafluoroethyl group, a pentafluoropropyl group, and a nonafluorobutyl group); and chloroalkyl groups (e.g., chloro(C₁₋₅)alkyl groups, such as a chloromethyl group).

Examples of the haloalkylene group include halo(C₁₋₅)alkylene groups, such as fluoroalkylene groups (e.g., perfluoro(C₁₋₅)alkylene groups, such as a tetrafluoroethylene group, a hexafluoropropane-1,3-diyl group, and an octafluorobutane-1,4-diyl group) and chloroalkylene groups (e.g., a perchloro(C₁₋₅)alkylene group).

The boron anion is, for example, preferably an anion represented by Formula (a-2) below. When the anion is used, a conductive material having a particularly low hygroscopicity can be formed, and corrosion of an easily corrodible layer, such as an electrode layer or a dielectric layer, caused by moisture content of the conductive material can be remarkably suppressed.

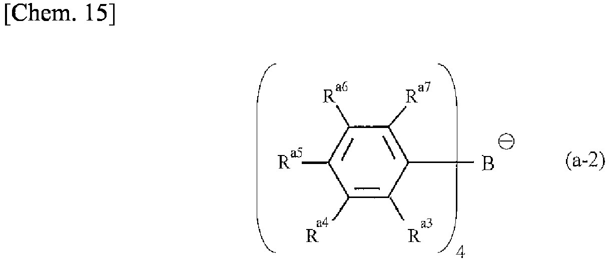

In the formula, R^{a3} to R^{a7} are identical or different and each represent a halogen atom or a haloalkyl group.

### First Electrode Layer

In a case where the electric or electronic device is a solid-state electrolytic capacitor, the first electrode layer is a positive electrode layer, and preferably a layer containing a valve metal. The positive electrode layer is, for example, made of valve metal foil, a sintered body containing valve metal particles, or the like. Examples of the valve metal include aluminum, tantalum, niobium, titanium, zirconium, hafnium, tungsten, and alloys containing these.

A surface of the layer containing the valve metal may be subjected to an area expansion treatment, such as etching.

### Dielectric Layer

In a case where the electric or electronic device is a solid-state electrolytic capacitor, a dielectric layer is preferably included between the positive electrode layer and the conductive material layer. The dielectric layer is, for example, a layer containing an oxide of a valve metal. In a case where the first electrode layer is a layer containing a valve metal, the dielectric layer can be formed by anodic oxidation of the first electrode layer. For example, in a case where aluminum foil is used in the first electrode layer, a dielectric layer containing an aluminum oxide on a surface of the first electrode layer can be formed by subjecting the first electrode layer to anodic oxidation.

### Second Electrode Layer

In a case where the electric or electronic device is a solid-state electrolytic capacitor, the second electrode layer is a negative electrode layer. The negative electrode layer is preferably a layer containing a metal, such as silver, and can be formed by, for example, applying and drying a resin composition containing silver particles to form a silver-containing resin film.

### Method for Manufacturing Electric or Electronic Device

The electric or electronic device can be produced through a process of doping a conjugated macromolecular compound with a dopant.

The method of doping a conjugated macromolecular compound with a dopant include a dry processing method and a wet processing method.

Examples of the dry processing method include a method of depositing a dopant to a surface of a conjugated macromolecular compound (e.g., a film, sheet, or coating film made of a conjugated macromolecular compound) by a vacuum vapor deposition method or a sputtering method.

An example of the wet processing method is a method, in which a conjugated macromolecular compound [or a layer containing a conjugated macromolecular compound (e.g., a film, sheet, or coating film made of a conjugated macromolecular compound)] is immersed in a dopant solution in which a dopant is dissolved and/or dispersed in a solvent or the dopant solution is applied onto a surface of a conjugated macromolecular compound [or a layer containing a conjugated macromolecular compound (e.g., a film, sheet, or coating film made of a conjugated macromolecular compound)], and then the resultant is dried. After drying, an annealing treatment may be performed as necessary.

The method for manufacturing the electric or electronic device by wet processing includes the following.
1: forming a layer containing a conjugated macromolecular compound by applying a solution containing the conjugated macromolecular compound to a substrate surface and drying the solution on the substrate surface; and
2: producing a conductive material layer containing a conductive material by applying a dopant solution to the layer containing the conjugated macromolecular compound and drying the dopant solution on the layer containing the conjugated macromolecular compound, the dopant solution containing a salt containing an anion and a counter cation, the anion being selected from the group consisting of a nitrogen anion, a boron anion, a phosphorus anion, and an antimony anion, and the conductive material having a configuration in which the conjugated macromolecular compound is doped with the anion.

### 1. Forming Layer Containing Conjugated Macromolecular Compound

A layer containing a conjugated macromolecular compound (or a thin film containing a conjugated macromolecular compound) is formed by applying a solution containing the conjugated macromolecular compound (hereinafter, may be referred to as "macromolecular compound solution") to a substrate surface and drying the solution on the substrate surface.

The method of forming the layer containing the conjugated macromolecular compound is not particularly limited and, for example, the layer may be formed by applying a macromolecular compound solution to a substrate or immersing a substrate into a macromolecular compound solution, and then volatilizing the solvent. The thickness of the coating film is, for example, approximately from 10 to 500 nm. The thickness of the coating film can be increased by multiple applications as necessary.

The substrate is not particularly limited. In a case where the electric or electronic device produces a solid-state electrolytic capacitor, a first electrode layer/dielectric layer layered body (specifically, an aluminum/aluminum oxide layered body) is preferably used as the substrate.

The macromolecular compound solution is a macromolecular compound solution containing at least the conjugated macromolecular compound described above and a solvent and, for example, can be produced by dissolving the conjugated macromolecular compound in the solvent.

The macromolecular compound solution may contain at least one conjugated macromolecular compound alone or may contain a combination of two or more conjugated macromolecular compounds.

The solvent is preferably a solvent having excellent solubility of a conjugated macromolecular compound, and examples thereof include aromatic hydrocarbons, such as benzene, toluene, xylene, ethylbenzene, trifluorotoluene, chlorobenzene, anisole, benzonitrile, nitrobenzene, and ethyl benzoate. One of these can be used alone or two or more in combination.

The macromolecular compound solution is preferable from the viewpoints that suppression of the moisture content can suppress the moisture content of the conductive material layer, and thus corrosion of an easily corrodible layer, such as an electrode layer or a dielectric layer, caused by moisture content of the conductive material can be suppressed. The moisture content is, for example, 2 wt.% or less, preferably 1 wt.% or less, more preferably 0.8 wt.% or less, even more preferably 0.7 wt.% or less, yet even more preferably 0.6 wt.% or less, particularly preferably 0.5 wt.% or less, most preferably 0.45 wt.% or less, and especially preferably 0.4 wt.% or less.

In a case where the macromolecular compound (1) or the macromolecular compound (2) described above is used as the conjugated macromolecular compound, these macromolecular compounds (1) and (2) each have a low hygroscopicity and excellent solubility in an organic solvent, and thus the moisture content in the macromolecular compound solution can be remarkably reduced.

In a case where a surface of a substrate (e.g., dielectric layer surface of a first electrode layer/dielectric layer layered body) is porous, or in a case where a surface of a substrate has protrusions and recesses, a contact surface area may be increased by, after formation of a layer containing the conjugated macromolecular compound, subjecting the layer to a heat treatment to soften the layer, and allowing the layer containing the conjugated macromolecular compound to suitably follow the surface of the substrate.

### 2. Producing Conductive Material Layer Containing Conductive Material

A conductive material layer containing a conductive material is produced by applying a dopant solution to the layer containing the conjugated macromolecular compound produced in the forming of a layer containing the conjugated macromolecular compound and drying the dopant solution on the layer containing the conjugated macromolecular compound, the dopant solution containing a salt containing an anion and a counter cation, the anion being selected from the group consisting of a nitrogen anion, a boron anion, a phosphorus anion, and an antimony anion, and the conductive material having a configuration in which the conjugated macromolecular compound is doped with the anion.

The dopant solution is a composition in which a dopant is dissolved and/or dispersed in a solvent. Note that one type of dopant may be contained alone, or a combination of two or more types of dopants may be contained.

Examples of the solvent include ester-based solvents, such as methyl acetate, ethyl acetate, isopropyl acetate, and butyl acetate; and nitrile-based solvents, such as acetonitrile, propionitrile, and benzonitrile. One of these can be used alone or two or more in combination.

Examples of the method of applying a dopant solution to a layer containing a conjugated macromolecular compound include a method of immersing a layer containing a conjugated macromolecular compound into a dopant solution. After application of the dopant solution and drying, an annealing treatment may be performed as necessary.

The moisture content of the dopant solution is preferably 1 wt.% or less, more preferably 0.8 wt.% or less, even more preferably 0.7 wt.% or less, yet even more preferably 0.6 wt.% or less, particularly preferably 0.5 wt.% or less, most preferably 0.45 wt.% or less, and especially preferably 0.4 wt.% or less. When the moisture content of the dopant solution is in the range described above, the moisture content of the conductive material layer can be suppressed, and thus corrosion of an easily corrodible layer, such as an electrode layer or a dielectric layer, caused by moisture content of the conductive material can be suppressed.

When a dopant described below, which has excellent solubility in the solvent described above, is used as the dopant, the moisture content in the dopant solution can be suppressed to the range described above.

When the conjugated macromolecular compound is doped with the dopant, the counter cation derived from a salt contained in the dopant exhibits a function as an oxidizer and withdraws an electron from the conjugated macromolecular compound. This creates holes serving as charge carriers in the conjugated macromolecular compound, and thus conductivity is exhibited. The valence of the counter cation that has withdrawn an electron decreases, and the counter cation is removed outside of the system of the conjugated macromolecular compound.

When the valence of the counter cation decreases, the anion derived from a salt contained in the dopant is released from the ionic bond of the counter cation. Then, the anion is stably placed in a gap of the crystalline structure of the conjugated macromolecular compound. Thus, the conjugated macromolecular compound achieves electrical conductivity, the crystallinity of the conjugated macromolecular compound is enhanced, and dedoping is suppressed.

The method for manufacturing an electric or electronic device may contain another processing as necessary and, for example, a second electrode layer may be formed on a surface of the conductive material layer after the conductive material layer containing the conductive material is produced.

In a case where the method for manufacturing an electric or electronic device is a method for manufacturing a solid-state electrolytic capacitor, a first electrode layer/dielectric layer layered body may be produced by forming an oxide film on a surface by subjecting valve metal foil to anodic oxidation prior to the forming of a layer containing a conjugated macromolecular compound, and a solution containing the conjugated macromolecular compound may be applied to a surface of the produced first electrode layer/dielectric layer layered body.

For the oxidation of the valve metal foil, the oxidation may be performed in the presence of oxygen, ozone, or a molecule that supplies an oxygen atom or in the presence of oxygen, ozone, or a molecule that supplies an oxygen atom, and light, plasma, or the like.

In a case where the method for manufacturing an electric or electronic device is a method for manufacturing a solid-state electrolytic capacitor, a graphite layer on a surface of the conductive material layer may be formed first and then a second electrode layer may be formed.

### Dopant

The dopant (e.g., p-type dopant) contains a salt containing an anion and a counter cation, the anion being selected from a nitrogen anion, a boron anion, a phosphorus anion, and an antimony anion.

The anion is as described in the section of "Anion" above.

The counter cation is not particularly limited as long as it is a cation that acts to withdraw an electron from the conjugated macromolecular compound, such as a metal cation (especially, alkali metal cation) such as a lithium cation, and a radical cation represented by Formula (b) below.

The radical cation represented by Formula (b) below is preferable from the viewpoints of having an excellent oxidizing power, readily withdrawing an electron from the conjugated macromolecular compound, and improving the doping amount of the anion. In particular, a compound represented by Formula (b) below, where at least one of R^{b1}, R^{b2}, and R^{b3} is a group represented by Formula (r) below, is preferred.

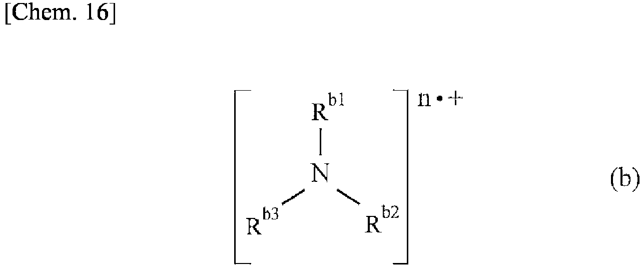

In Formula (b), R^{b1} to R^{b3} are identical or different and each represent a monovalent aromatic group or a group represented by Formula (r) below. n represents valence of the radical cation and is equal to the number (n) of nitrogen atoms in Formula (b).

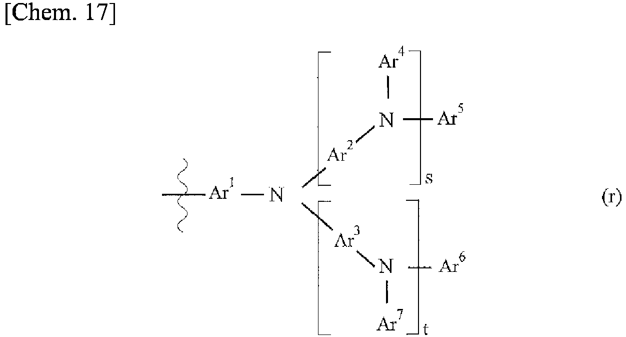

In Formula (r), Ar¹, Ar², and Ar³ are identical or different and each represent a divalent aromatic group; Ar⁴, Ar⁵, Ar⁶, and Ar⁷ are identical or different and each represent a monovalent aromatic group which may have a substituent represented by Formula (sb) below. s and t are identical or different, and each represent an integer of 0 or greater. A bond indicated by a wavy line in the formula is bonded to the nitrogen atom in Formula (b).

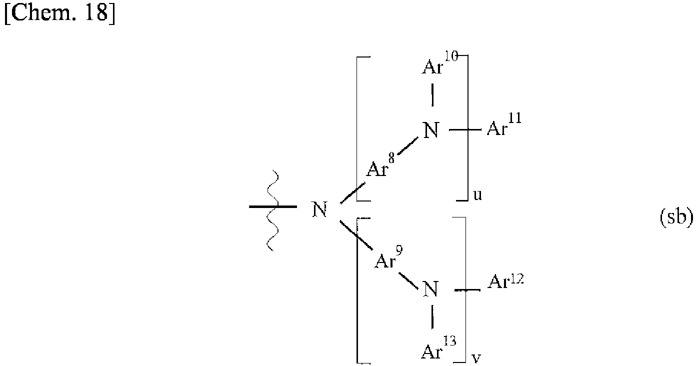

In Formula (sb), Ar⁸ and Ar⁹ are identical or different and each represent a divalent aromatic group. Ar¹⁰, Ar¹¹, Ar¹², and Ar¹³ are identical or different and each represent a monovalent aromatic group. u and v are identical or different and each represent an integer of 0 or greater. A bond indicated by a wavy line in Formula (sb) is bonded to a monovalent aromatic group in Formula (r).

s, t, u, and v each represent an integer of 0 or greater, and are, for example, from 0 to 5, preferably from 0 to 3, and particularly preferably from 0 to 2. As the values of s, t, u, and v increase, the doping efficiency with respect to the conjugated macromolecular compound tends to improve.

When s, t, u, and v are each an integer of 2 or greater, there are a plurality of groups that are put in parentheses. These groups may be identical or different.

The monovalent aromatic group is a group having the structural formula of the aromatic compound with one hydrogen atom removed [more specifically, an aromatic group with one hydrogen atom removed, the hydrogen atom binding to a carbon atom that constitutes the aromatic compound (a carbon atom or a heteroatom that constitutes the aromatic compound, in a case where the aromatic compound is an aromatic heterocycle)].

Also, the divalent aromatic group is a group having a structural formula of the aromatic compound with two hydrogen atoms removed [more specifically, an aromatic group with two hydrogen atoms removed, the hydrogen atoms binding to a carbon atom that constitutes the aromatic compound (a carbon atom or a heteroatom that constitutes the aromatic compound, in a case where the aromatic compound is an aromatic heterocycle)].

The aromatic compound includes aromatic hydrocarbons and aromatic heterocycles.

Examples of the aromatic hydrocarbons include aromatic hydrocarbon rings having from 6 to 14 carbons such as benzene and naphthalene, and structural bodies in which two or more of the aromatic hydrocarbon rings are bonded through a single bond or a linking group.

Examples of the linking group include a C₁₋₅ alkylene group, a carbonyl group (-CO-), an ether bond (-O-), a thioether bond (-S-), an ester bond (-COO-), an amide bond (-CONH-), and a carbonate bond (-OCOO-).

Among them, the aromatic hydrocarbon is preferably at least one selected from aromatic hydrocarbons represented by Formulas (ar-1) to (ar-6) below.

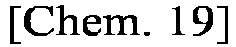

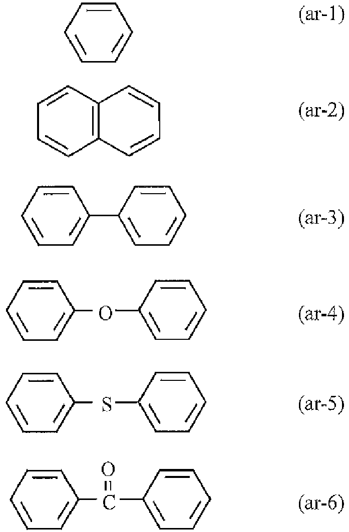

Examples of the aromatic heterocycle include monocyclic aromatic heterocycles having a carbon atom and at least one heteroatom (e.g., an oxygen atom, a sulfur atom, a nitrogen atom, a phosphorus atom, or the like) as an atom constituting the ring, and fused rings in which one or two or more aromatic hydrocarbon rings are fused with the monocyclic aromatic heterocycles. Specific examples include pyrrole, furan, thiophene, phosphole, pyrazole, imidazole, oxazole, isoxazole, thiazole, indole, benzofuran, benzothiophene, isoindole, isobenzofuran, benzophosphole, benzimidazole, benzoxazole, benzothiazole, benzisoxazole, indazole, benzisothiazole, benzotriazole, purine, pyridine, phosphinine, pyrimidine, pyrazine, pyridazine, triazine, 1,2,4,5-tetrazine, 1,2,3,4-tetrazine, 1,2,3,5-tetrazine, hexazine, quinoline, isoquinoline, quinoxaline, quinazoline, cinnoline, pteridine, phthalazine, acridine, 4aH-phenoxazine, and carbazole.

The monovalent aromatic group and the divalent aromatic group may have a substituent. Examples of the substituent include a halogen atom, a C₁₋₅ alkyl group, an oxo group, a hydroxyl group, a substituted oxy group (for example, a C₁₋₅ alkoxy group and a C₁₋₅ acyloxy group), a carboxyl group, a substituted oxycarbonyl group (for example, a C₁₋₅ alkoxycarbonyl group), a substituted or unsubstituted carbamoyl group, a cyano group, a nitro group, an amino group, and a substituted amino group (for example, a mono- or di-C₁₋₅ alkylamino group, and a mono- or di-C₁₋₅ acylamino group). Among the substituents, a halogen atom is preferred, and a bromine atom is particularly preferred.

Furthermore, the monovalent aromatic group may have, in addition to the substituent described above, a group represented by the following Formula (sb-1), for example.

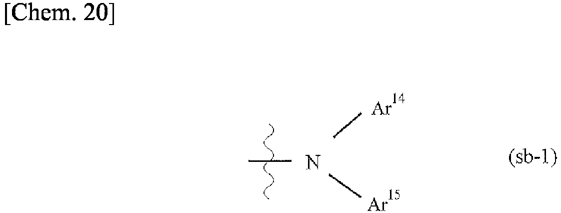

In the formula, Ar¹⁴ and Ar¹⁵ are identical or different, and each represent a monovalent aromatic group. A bond indicated by a wavy line in the formula is bonded to a carbon atom constituting the aromatic compound (in a case where the aromatic compound is an aromatic heterocycle, a carbon atom or a heteroatom).

Examples of the monovalent aromatic group in Ar¹⁴ and Ar¹⁵ in the formula above include the same examples as described above.

The dopant may contain a component other than the salt containing the anion and the counter cation and, for example, may contain an oxidizer. The oxidizer acts to withdraw an electron from the conjugated macromolecular compound and exhibits an effect of improving the doping amount of the anion.

In particular, in a case where the dopant contains the salt containing the anion and the metal cation (especially, alkali metal cation) such as a lithium cation, an oxidizer is preferably contained together with the salt.

Examples of the oxidizer include α,β-unsaturated nitrile compound having a conjugated double bond structure, NOPF₆, iron trichloride (FeCl₃), and simple halogen substances (e.g., iodine I₂, bromine Br₂, and chlorine Cl₂). One of these can be used alone or two or more in combination.

Examples of the α,β-unsaturated nitrile compound having a conjugated double bond structure include compounds represented by Formulas (4-1) to (4-4) below.

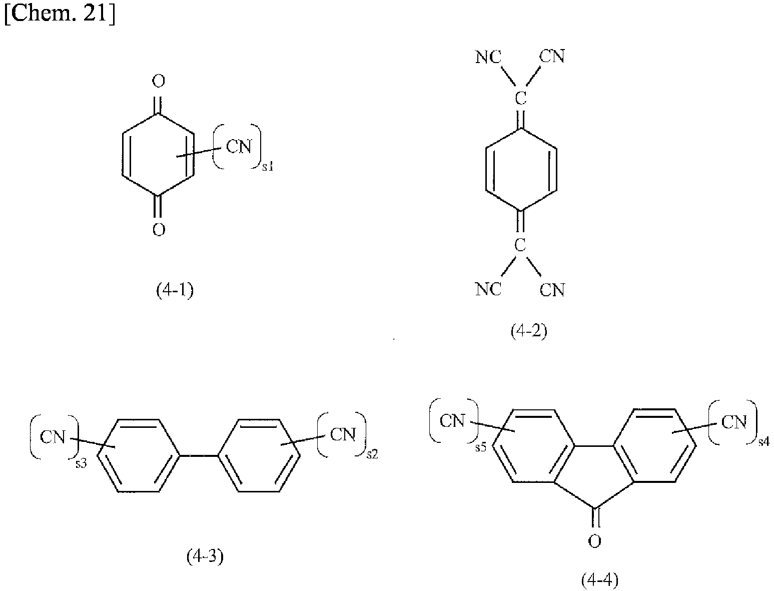

In the formulas above, s1 to s5 each represent the number of cyano groups bonded to a ring structure, are identical or different, and each are an integer of 1 or greater. To a ring structure in the formulas, a substituent other than a cyano group (e.g., halogen atom) may be bonded.

The total number of the cyano groups contained in the compound is 1 or greater, and preferably 2 or greater. Note that the upper limit of the total number of the cyano groups is, for example, 4.

Specific examples of the α,β-unsaturated nitrile compound having a conjugated double bond structure include tetrafluorotetracyanoquinodimethane, 2,3-dichloro-5,6-dicyano-1,4-benzoquinone, 2,3-diiodo-5,6-dicyanobenzoquinone, 2,2',4,4'-tetracyanobiphenol, 2,4,7-tricyano-9-fluorene, tetracyanoquinodimethane, 2,5-difluoro-7,7,8,8-tetracyanoquinodimethane, and 2-fluoro-7,7,8,8-tetracyanoquinodimethane.

An amount of the oxidizer to be used is, for example, approximately from 0.5 to 5 mol with respect to 1 mol of the salt containing the anion and the counter cation.

### Method for Producing Salt Containing Anion and Counter Cation

For example, the salt containing the anion represented by Formula (a-1) or (a-2) above and the radical cation represented by Formula (b) above can be produced through reacting an ionic compound containing an anion represented by Formula (a-1) or (a-2) and an amine compound corresponding to the radical cation represented by Formula (b) above in the presence of an oxidizer.

The ionic compound and the amine compound each can be used alone or two or more in combination.

Examples of the ionic compound include an ionic compound containing a nitrogen anion or a boron anion and a monovalent metal ion. Examples of the metal ion include alkali metal ions such as Li⁺ and Na⁺; alkaline earth metal ions such as Mg²⁺ and Ca²⁺; and transition metal ions such as Cu⁺, Ag⁺, and Au⁺.

The ionic compound is, for example, preferably a monovalent metal salt of TFSI⁻ such as silver bis(trifluoromethanesulfonyl)imide (AgTFSI).

Examples of the amine compound include tris(halophenyl)amine such as tris(p-bromophenyl)amine.

An amount of the ionic compound used is, for example, approximately from 1 to 5 mol per mole of the amine compound.

Examples of the oxidizer include NOPF₆, iron trichloride (FeCl₃), and simple halogen substances (e.g., iodine I₂, bromine Br₂, and chlorine Cl₂). One of these can be used alone or two or more in combination.

An amount of the oxidizer used is, for example, approximately from 1 to 5 mol per mole of the amine compound.

This reaction can be performed in the presence of a solvent. Examples of the solvent include nitrile-based solvents such as acetonitrile, propionitrile, and benzonitrile; water; alcohol-based solvent such as methanol; amide-based solvents such as N,N-dimethylformamide and N,N-dimethylacetamide; ether based solvents such as diethyl ether, THF, and dioxane; and ester-based solvents such as ethyl acetate. One of these can be used alone or two or more in combination.

A reaction atmosphere may be any atmosphere that does not inhibit the reaction and is not particularly limited, and may be, for example, any of an air atmosphere, a nitrogen atmosphere, and an argon atmosphere.

A temperature of the reaction is, for example, approximately from -70 to 60°C. A reaction time is, for example, approximately from 0.5 to 5 hours.

After completion of the reaction, the resulting reaction product can be separated and purified by a separation means, such as filtration, concentration, distillation, extraction, crystallization, adsorption, recrystallization, or column chromatography; or a separation means in combination of these.

In a case where a salt of the anion represented by Formula (a-1) or (a-2) above and a radical cation, which is represented by Formula (b) and in which at least one of R^{b1} to R^{b3} in Formula (b) is a group represented by Formula (r), (hereinafter, may be referred to as "radical cation (b')") is produced, an amine compound corresponding to the radical cation (b') is used in place of an amine compound corresponding to the radical cation (b) in the reaction above.

The amine compound corresponding to the radical cation (b') can be produced, for example, by subjecting a halide of a triarylamine to a coupling reaction (e.g., Sholl coupling reaction) using an oxidizer such as 2,3-dichloro-5,6-dicyano-1,4-benzoquinone (DDQ).

### Electric or Electronic Apparatus

The electric or electronic apparatus of the present disclosure includes the electric or electronic device described above. The electric or electronic apparatus preferably includes an electrolytic capacitor having a configuration including a first electrode layer, a conductive material layer, and a second electrode layer that are layered in this order (e.g., solid-state electrolytic capacitor), an organic EL element, or an organic solar cell (e.g., organic thin-film solar cell) described above.

Because the electric or electronic apparatus includes the conductive material layer described above (i.e., conductive material layer having heat resistance and moisture resistance), excellent electrical characteristics can be stably exhibited for a long period of time even in a high temperature and high humidity environment.

Examples of the electric or electronic apparatus include a mobile device, such as a cellular phone, a notebook computer, a handheld game console, and a wearable device (e.g., including a watch-type, eyewear-type, or earphone-type wearable device); a multimedia device, such as a television; a lighting device, such as an organic EL lighting device; a display device, such as an organic EL display; a device for an automobile, such as car navigation equipment and an engine control circuit; and a photovoltaic power generation panel, a solar energy system, and a battery charger for a mobile terminal.

Each of the configurations, their combinations, and the like of the present disclosure above is an example, and addition, omission, substitution, and change of the configuration can be appropriately made without departing from the gist of the present disclosure.

### Examples

Hereinafter, the present disclosure will be described more specifically with reference to examples, but the present disclosure is not limited by these examples, and is limited only by the claims.

### Preparation Example 1 (Preparation of Dopant)

Silver bis(trifluoromethanesulfonyl)imide (AgTFSI, 3.51 mmol), tris(4-bromophenyl)amine (2.51 mmol), and diethyl ether (100 mL) were mixed and stirred for 20 minutes. Thereafter, the mixture was cooled to -36°C.

A mixed solution of iodine (2.51 mmol)/diethyl ether (30 mL) was added dropwise to the mixture over 15 minutes, and then the temperature was raised to room temperature. A precipitate was collected by filtration and dried under reduced pressure at room temperature for 2 hours to produce a dopant (1) represented by the following formula.

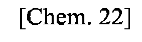

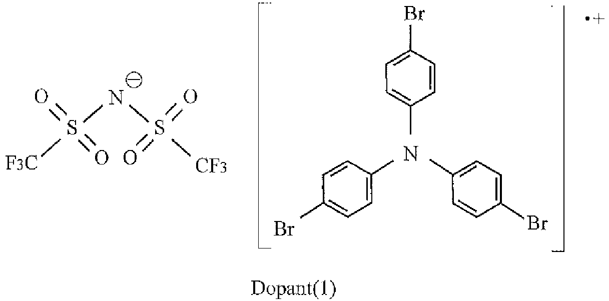

### Preparation Example 2 (Preparation of Dopant)

Lithium bis(trifluoromethanesulfonyl)imide (LiTFSI, 0.2 mmol), 2,3,5,6-tetrafluorotetracyanoquinodimethane (0.2 mmol), and butyl acetate (10 mL) as a solvent were mixed and stirred for 3 minutes. This resulted in a dopant (2).

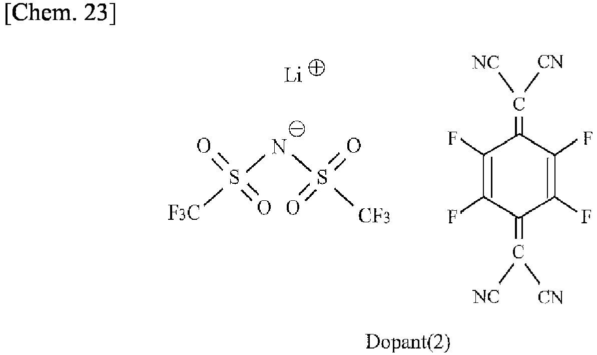

### Preparation Example 3 (Preparation of Dopant)

Lithium bis(trifluoromethanesulfonyl)imide (LiTFSI, 0.2 mmol), 2,3-dichloro-5,6-dicyanobenzoquinone (0.2 mmol), and acetonitrile (10 mL) as a solvent were mixed and stirred for 3 minutes. This resulted in a dopant (3).

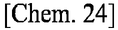

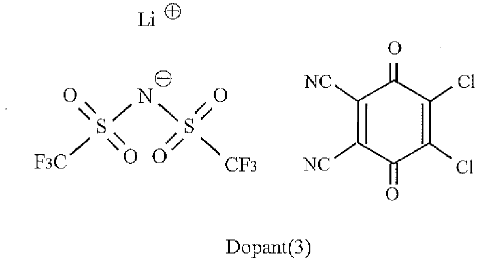

### Preparation Example 4 (Preparation of Macromolecular Compound Having Electron Donating Group Containing Heteroatom in Side Chain)

In the presence of toluene in an amount to achieve a concentration of 0.5 M against a substrate, with 5 mol% of p-toluenesulfonic acid-monohydrate with respect to the substrate, 1.1 mol equivalent of 1-tridecanol was reacted with 3-methoxythiophene as the substrate under reflux for 4 hours to produce 3-tridecyloxythiophene (yield: 92%).

In the presence of THF in an amount to achieve a concentration of 0.5 M against a substrate, 1.0 mol equivalent of NBS was reacted with 3-tridecyloxythiophene as the substrate at 0°C for 30 minutes to produce 2-bromo-4-tridecyloxythiophene (yield: 97%).

In the presence of THF in an amount to achieve a concentration of 0.1 M against a substrate, 1 mol equivalent of lithium diisopropylamide and 1 mol equivalent of copper (II) chloride were mixed with 2-bromo-4-tridecyloxythiophene as the substrate at -78°C, and then the temperature was raised to 25°C. Then, they were reacted for 12 hours while the temperature was maintained at 25°C to produce 5,5'-dibromo-3,3'-tridecyloxy-2,2'-bithiophene (yield: 73%).

In the presence of chlorobenzene in an amount to achieve a concentration of 0.05 M against a substrate, with 2.0 mol% of Pd₂(dba)₃·CHCl₃ with respect to the substrate, and 8.0 mol% of tri(o-tolyl)phosphine with respect to the substrate, 5,5'-dibromo-3,3'-tridecyloxy-2,2'-bithiophene as the substrate was reacted with 2,5-bis(trimethylstannyl)thieno[3,2-b]thiophene under microwave irradiation. By this, a macromolecular compound (1-1-2a) containing a repeating unit represented by Formula (1-1-2a) below was produced (ionization potential determined by photoemission yield spectroscopy: 4.5 eV).

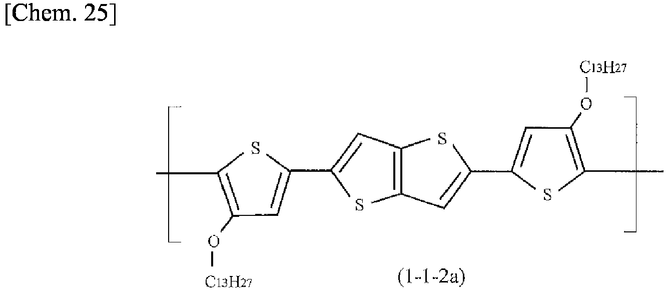

### Example 1

### First Electrode Layer/Dielectric Layer Layered Body Formation

As a positive electrode conductor containing a valve metal, a porous aluminum was used. By subjecting the aluminum to anodic oxidation, an oxide film was formed on a surface of the aluminum, and thus an aluminum/aluminum oxide layered body was produced.

### Conductive Material Layer Formation-1: Formation of Layer Containing Conjugated Macromolecular Compound

Next, the aluminum/aluminum oxide layered body was immersed in a toluene solution of a macromolecular compound (1-1-2a) produced in Preparation Example 4 (moisture content: 0.3 wt.%) and taken out. Thereafter, this was dried at 80°C and solidified, and thus an aluminum/aluminum oxide/macromolecular compound thin film layered body was formed.

### Conductive Material Layer Formation-2: Doping

Thereafter, the aluminum/aluminum oxide/macromolecular compound thin film layered body was immersed in the butyl acetate solution of the dopant (1) produced in Preparation Example 1 (moisture content: 0.3 wt.%) and then dried at 80°C, then washed with butyl acetate, and dried at 80°C. By this, an aluminum oxide/aluminum oxide/conductive material layer layered body was formed.

### Second Electrode Layer Formation

On the conductive material layer of the produced layered body, a graphite layer and a silver-containing resin layer were formed in this order. By this, a solid-state electrolytic capacitor (1) [aluminum/aluminum oxide/conductive material layer/graphite layer/silver-containing resin layer] was produced.

It was confirmed that the produced solid-state electrolytic capacitor (1) functioned as a capacitor by applying a voltage in a range from -9 V to 9 V by using a direct-current power source at 25°C and measuring a current value after 60 seconds at each voltage.

### Low ESR Characteristic Evaluation

For the produced solid-state electrolytic capacitor (1), the ESR value was measured at 25°C and at a frequency of 100 kHz by using an LCR meter. Note that, for the ESR value, a total negative electrode area was normalized to a unit area (1 cm²). The results are presented in Table 1.

### Evaluation of Heat Resistance

The solid-state electrolytic capacitor after undergoing the heat resistance test at 150°C for 2000 hours was subjected to ESR value measurement in the same manner as for the "Low ESR Characteristic Evaluation", and an increase percentage from the ESR value at 25°C was calculated. The results are presented in Table 1.

### Example 2

A solid-state electrolytic capacitor (2) was produced in the same manner as in Example 1 except for using a 1,2-dichlorobenzene solution (moisture content: 0.3 wt.%) of the macromolecular compound (1-1-2a) in place of the toluene solution of the macromolecular compound (1-1-2a) in performing the "Conductive Material Layer Formation-1", and using a butyl acetate solution of the dopant (2) produced in Preparation Example 2 (moisture content: 0.3 wt.%) in place of the butyl acetate solution of the dopant (1) in performing the "Conductive Material Layer Formation-2". The low ESR characteristics and heat resistance were evaluated in the same manner as in Example 1.

When the dopant (2) is used, the 2,3,5,6-tetrafluorotetracyanoquinodimethane functions as an oxidizer for a macromolecular compound, and the TFSI anion is introduced into the macromolecular compound as the counter anion for the oxidized macromolecular compound. At this time, the 2,3,5,6-tetrafluorotetracyanoquinodimethane becomes a radical anion, is dissolved in the butyl acetate together with the Li cation, and is removed from the system of the macromolecular compound.

### Example 3

A solid-state electrolytic capacitor (3) was produced in the same manner as in Example 1 except for using an acetonitrile solution of the dopant (3) produced in Preparation Example 3 (moisture content: 0.3 wt.%) in place of the butyl acetate solution of the dopant (1) in performing the "Conductive Material Layer Formation-2". The low ESR characteristics and heat resistance were evaluated in the same manner as in Example 1.

When the dopant (3) is used, the 3-dichloro-5,6-dicyanobenzoquinone functions as an oxidizer for a macromolecular compound, and the TFSI anion is introduced into the macromolecular compound as the counter anion for the oxidized macromolecular compound. At this time, the 2,3-dichloro-5,6-dicyanobenzoquinone becomes a radical anion, is dissolved in the acetonitrile together with the Li cation, and is released from the system of the macromolecular compound.

### Comparative Example 1

A solid-state electrolytic capacitor (4) was produced in the same manner as in Example 1 except for performing the following "Conductive Material Layer Formation" in place of the "Conductive material layer formation-1" and "Conductive material layer formation-2". The low ESR characteristics and heat resistance were evaluated in the same manner as in Example 1.

### Conductive Material Layer Formation

The aluminum/aluminum oxide layered body was immersed in an aqueous PEDOT/PSS dispersion and taken out. Thereafter, this was dried at 125°C and solidified, and thus a conductive material layer was formed.

### [Table 1]

**Table 1**

| | ESR (mΩ/cm²) | | ESR increase percentage (%) |
|---|---|---|---|
| | Before heat resistance test 25°C | After heat resistance test 150°C × 2000 hrs | |
| Example 1 | 1.7 | 2.1 | 23.5 |
| Example 2 | 1.9 | 2.3 | 21.0 |
| Example 3 | 2.1 | 2.7 | 28.6 |
| Comparative Example 1 | 3.9 | 5.8 | 48.7 |

### Example 4

A solid-state electrolytic capacitor (5) was produced by the same method as in Example 1, that is, by using the toluene solution (moisture content: 0.3 wt.%) of the macromolecular compound (1-1-2a) in performing the "Conductive Material Layer Formation-1", and using the butyl acetate solution (moisture content: 0.3 wt.%) of the dopant (1) in performing the "Conductive Material Layer Formation-2".

### Moisture Resistance Evaluation

For the produced solid-state electrolytic capacitor (5), the ESR value was measured at 25°C and at a frequency of 120 kHz by using an LCR meter. Note that, for the ESR value, a total negative electrode area was normalized to a unit area (1 cm²).

Thereafter, the produced solid-state electrolytic capacitor (5) was subjected to a moisture resistance test in which the solid-state electrolytic capacitor (5) was allowed to stand at 25°C and 85%RH for 3 days.

For the solid-state electrolytic capacitor after the moisture resistance test, the ESR value was measured at 25°C and at a frequency of 120 kHz by using an LCR meter.

Then, the ESR increase percentage by the moisture resistance test was calculated. The results are listed in Table 2.

### Example 5

A solid-state electrolytic capacitor (6) was produced in the same manner as in Example 1 except for using the toluene solution (moisture content: 0.5 wt.%) of the macromolecular compound (1-1-2a) in performing the "Conductive Material Layer Formation-1", and using the butyl acetate solution (moisture content: 0.5 wt.%) of the dopant (1) in performing the "Conductive Material Layer Formation-2". The moisture resistance of the produced solid-state electrolytic capacitor was evaluated by the same method as in Example 4.

### Example 6

A solid-state electrolytic capacitor (7) was produced in the same manner as in Example 1 except for using the toluene solution (moisture content: 0.95 wt.%) of the macromolecular compound (1-1-2a) in performing the "Conductive Material Layer Formation-1", and using the butyl acetate solution (moisture content: 0.95 wt.%) of the dopant (1) in performing the "Conductive Material Layer Formation-2". The moisture resistance of the produced solid-state electrolytic capacitor was evaluated by the same method as in Example 4.

### Example 7

A solid-state electrolytic capacitor (8) was produced in the same manner as in Example 1 except for using the toluene solution (moisture content: 1.1 wt.%) of the macromolecular compound (1-1-2a) in performing the "Conductive Material Layer Formation-1", and using the butyl acetate solution (moisture content: 0.3 wt.%) of the dopant (1) in performing the "Conductive Material Layer Formation-2". The moisture resistance of the produced solid-state electrolytic capacitor was evaluated by the same method as in Example 4.

### Example 8

A solid-state electrolytic capacitor (9) was produced in the same manner as in Example 1 except for using the toluene solution (moisture content: 1.1 wt.%) of the macromolecular compound (1-1-2a) in performing the "Conductive Material Layer Formation-1", and using the butyl acetate solution (moisture content: 1.1 wt.%) of the dopant (1) in performing the "Conductive Material Layer Formation-2". The moisture resistance of the produced solid-state electrolytic capacitor was evaluated by the same method as in Example 4.

### Comparative Example 2

A solid-state electrolytic capacitor (10) was produced by the same method as in Comparative Example 1. The moisture resistance of the produced solid-state electrolytic capacitor was evaluated by the same method as in Example 4.

### [Table 2]

**Table 2**

| | ESR (mΩ/cm²) | | ESR increase percentage (%) |
|---|---|---|---|
| | Before moisture resistance test 25°C | After moisture resistance test 25°C, 85%RH × 3 days | |
| Example 4 | 0.38 | 0.39 | 2.6 |
| Example 5 | 0.38 | 0.41 | 7.9 |
| Example 6 | 0.38 | 0.42 | 10.5 |
| Example 7 | 0.38 | 0.45 | 18.4 |
| Example 8 | 0.38 | 0.55 | 44.7 |
| Comparative Example 2 | 0.39 | 0.97 | 148.7 |

As is clear from Table 2, when the essential water content is suppressed in performing the formation of the Conductive Material layer, the increase percentage of the ESR value can be remarkably suppressed even in a high humidity environment.

As a summary of the above, configurations and variations of the present disclosure are described below.
[1] An electric or electronic device having a configuration including a first electrode layer, a conductive material layer, and a second electrode layer that are layered in this order,
   the conductive material layer containing a conductive material having a configuration in which a conjugated macromolecular compound is doped with an anion selected from the group consisting of a nitrogen anion, a boron anion, a phosphorus anion, and an antimony anion.
[2] The electric or electronic device according to [1], in which the anion is an anion represented by Formula (a-1) or (a-2).
[3] The electric or electronic device according to [1] or [2], in which an ionization potential of the conjugated macromolecular compound is 6.0 eV or less.
[4] The electric or electronic device according to any one of [1] to [3], in which the conjugated macromolecular compound is a macromolecular compound containing at least one selected from the group consisting of repeating units represented by Formulas (1a) to (1c) or a macromolecular compound containing a repeating unit represented by Formula (2).
[4] The electric or electronic device according to [3], in which the macromolecular compound containing at least one selected from the group consisting of repeating units represented by Formulas (1a) to (1c) is a macromolecular compound containing a repeating unit represented by Formula (1-1).
[5] The electric or electronic device according to [4], in which at least one of D¹¹ to D¹⁶ in Formula (1-1) is a group represented by Formula (d-1) or (d-2).
[6] The electric or electronic device according to [3], in which the macromolecular compound containing at least one selected from the group consisting of repeating units represented by Formulas (1a) to (1c) is a macromolecular compound containing a repeating unit represented by Formula (1-1-1) or a repeating unit represented by Formula (1-1-2).
[7] The electric or electronic device according to [3], in which the macromolecular compound containing at least one selected from the group consisting of repeating units represented by Formulas (1a) to (1c) is a macromolecular compound containing a repeating unit represented by Formula (1-1-2).
[8] The electric or electronic device according to [1] or [2], in which the conjugated macromolecular compound is a macromolecular compound containing a repeating unit represented by Formula (1-1).
[9] the conjugated macromolecular compound is a macromolecular compound containing a repeating unit represented by Formula (1-1-1) or a repeating unit represented by Formula (1-1-2),
[10] The electric or electronic device according to [1] or [2], in which the conjugated macromolecular compound is a macromolecular compound containing a repeating unit represented by Formula (1-1-2).
[11] The electric or electronic device according to any one of [6] to [10], in which at least one of D¹¹ to D¹⁶ in the formula is a group represented by Formula (d-1) or (d-2).
[12] The electric or electronic device according to any one of [1] to [11], in which a moisture absorption amount determined by a moisture resistance test described below of the conductive material layer is 1 wt.% or less:
   Moisture resistance test: a test performed by allowing the conductive material layer to stand at 25°C and 85%RH for 3 days.
[13] The electric or electronic device according to any one of [1] to [11], in which an electric conductivity of the conductive material layer is 1 S/cm or greater.
[14] The electric or electronic device according to any one of [1] to [13], in which the electric or electronic device is an electrolytic capacitor, an organic electroluminescent element, or an organic solar cell.
[15] A method for manufacturing an electric or electronic device described in any one of [1] to [14], the method including:
   doping a conjugated macromolecular compound with a dopant.
[16] A method for manufacturing an electric or electronic device described in any one of [1] to [14], the method including:
   1: forming a layer containing a conjugated macromolecular compound by applying a solution containing the conjugated macromolecular compound to a substrate surface and drying the solution on the substrate surface; and
   2: producing a conductive material layer containing a conductive material by applying a dopant solution to the layer containing the conjugated macromolecular compound and drying the dopant solution on the layer containing the conjugated macromolecular compound, the dopant solution containing a salt containing an anion and a counter cation, the anion being selected from the group consisting of a nitrogen anion, a boron anion, a phosphorus anion, and an antimony anion, and the conductive material having a configuration in which the conjugated macromolecular compound is doped with the anion.
[17] The method for manufacturing an electric or electronic device according to [16], in which a moisture content of the solution containing the conjugated macromolecular compound is 2 wt.% or less.
[18] The method for manufacturing an electric or electronic device according to [16] or [17], in which a moisture content of the dopant solution is 1 wt.% or less.
[19] The method for manufacturing an electric or electronic device according to any one of [16] to [18], in which the dopant solution contains an oxidizer and a salt containing an anion and a counter cation, the anion being selected from a nitrogen anion, a boron anion, a phosphorus anion, and an antimony anion, and a content of the oxidizer is from 0.5 to 5 mol with respect to 1 mol of the salt.
[20] The method for manufacturing an electric or electronic device according to [19], in which the oxidizer is an α,β-unsaturated nitrile compound having a conjugated double bond structure.
[20] The method for manufacturing an electric or electronic device according to [19], in which the oxidizer is at least one compound selected from the group consisting of compounds represented by Formulas (4-1) to (4-4).
[21] The method for manufacturing an electric or electronic device according to any one of [16] to [20], in which the salt is a salt containing an anion and a metal cation, the anion being selected from a nitrogen anion, a boron anion, a phosphorus anion, and an antimony anion.
[22] The method for manufacturing an electric or electronic device according to any one of [16] to [18], in which the salt is a salt containing an anion and a radical cation represented by Formula (b), the anion being selected from a nitrogen anion, a boron anion, a phosphorus anion, and an antimony anion.
[23] An electric or electronic apparatus including the electric or electronic device described in any one of [1] to [14].

### Industrial Applicability

The electric or electronic device of the present disclosure has excellent heat resistance. Furthermore, corrosion of an easily corrodible layer, such as an electrode layer or a dielectric layer, can be prevented even in a high humidity environment, and high electrical characteristics can be maintained. Thus, the electric or electronic device can be suitably used for a purpose requiring moisture resistance and heat resistance.

## Claims

1. An electric or electronic device having a configuration comprising a first electrode layer, a conductive material layer, and a second electrode layer that are layered in this order,
the conductive material layer containing a conductive material having a configuration in which a conjugated macromolecular compound is doped with an anion selected from the group consisting of a nitrogen anion, a boron anion, a phosphorus anion, and an antimony anion.

2. The electric or electronic device according to claim 1, wherein the anion is an anion represented by Formula (a-1) or (a-2): 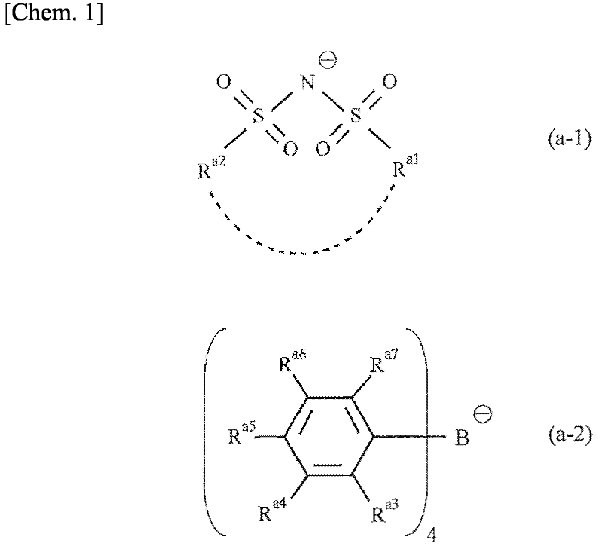 where R^{a1} to R^{a7} are identical or different and each represent a halogen atom or a haloalkyl group; and R^{a1} and R^{a2} may be bonded to each other to form a haloalkylene group.

3. The electric or electronic device according to claim 1 or 2, wherein the conjugated macromolecular compound is a macromolecular compound containing at least one selected from the group consisting of repeating units represented by Formulas (1a) to (1c) or a macromolecular compound containing a repeating unit represented by Formula (2): 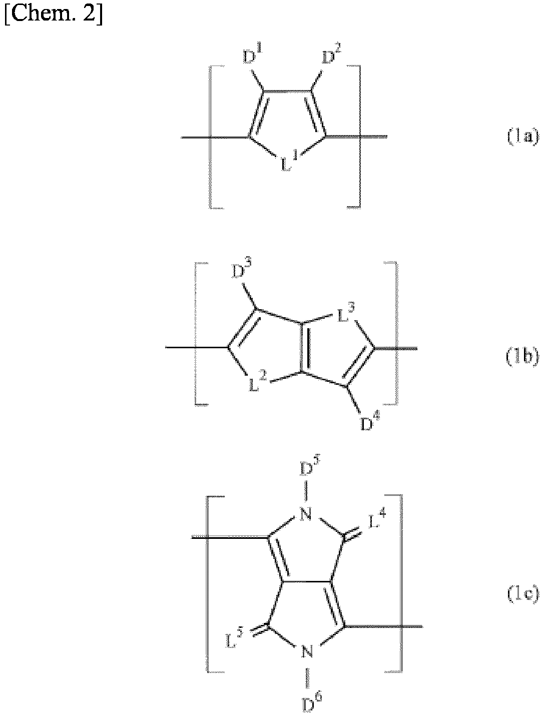 where L¹ to L⁵ are identical or different and each represent an element of Groups 13 to 16; and D¹ to D⁶ are identical or different and each represent a group selected from the group consisting of an alkyl group, a haloalkyl group, an electron donating group containing a heteroatom, and a hydrogen atom, with proviso that at least one selected from the group consisting of D¹ to D⁶ is a group selected from the group consisting of an alkyl group, a haloalkyl group, and an electron donating group containing a heteroatom; 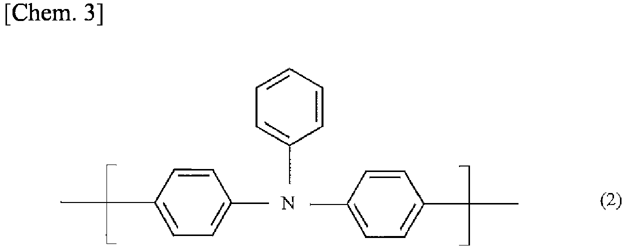 where a benzene ring may contain a substituent.

4. The electric or electronic device according to claim 3, wherein the macromolecular compound containing at least one selected from the group consisting of repeating units represented by Formulas (1a) to (1c) is a macromolecular compound containing a repeating unit represented by Formula (1-1): 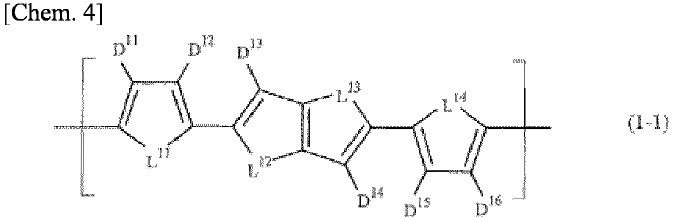 where L¹¹ to L¹⁴ are identical or different and each represent an element of Groups 13 to 16; and D¹¹ to D¹⁶ are identical or different and each represent a group selected from the group consisting of an alkyl group, a haloalkyl group, an electron donating group containing a heteroatom, and a hydrogen atom, with proviso that at least one selected from the group consisting of D¹¹ to D¹⁶ is a group selected from the group consisting of an alkyl group, a haloalkyl group, and an electron donating group containing a heteroatom.

5. The electric or electronic device according to claim 4, wherein at least one of D¹¹ to D¹⁶ in Formula (1-1) is a group represented by Formula (d-1) or (d-2): 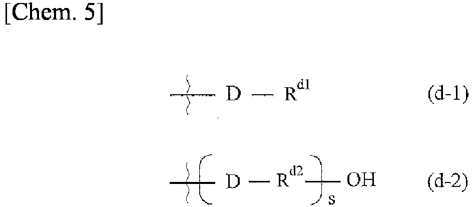 where D represents at least one heteroatom selected from the group consisting of elements of Groups 14 to 16; R^{d1} represents an aliphatic hydrocarbon group having from 5 to 30 carbons; R^{d2} represents an aliphatic hydrocarbon group having from 1 to 5 carbons, and s represents an integer of 1 or greater; and a bond indicated by a wavy line in the formulas is bonded to a main chain of the macromolecular compound containing a repeating unit represented by Formula (1-1).

6. The electric or electronic device according to claim 1 or 2, wherein the electric or electronic device is an electrolytic capacitor, an organic electroluminescent element, or an organic solar cell.

7. A method for manufacturing an electric or electronic device described in claim 1 or 2, the method comprising:
doping a conjugated macromolecular compound with a dopant.

8. A method for manufacturing an electric or electronic device described in claim 1 or 2,
the method comprising:
1: forming a layer containing a conjugated macromolecular compound by applying a solution containing the conjugated macromolecular compound to a substrate surface and drying the solution on the substrate surface; and
2: producing a conductive material layer containing a conductive material by applying a dopant solution to the layer containing the conjugated macromolecular compound and drying the dopant solution on the layer containing the conjugated macromolecular compound, the dopant solution containing a salt containing an anion and a counter cation, the anion being selected from the group consisting of a nitrogen anion, a boron anion, a phosphorus anion, and an antimony anion, and the conductive material having a configuration in which the conjugated macromolecular compound is doped with the anion.

9. The method for manufacturing an electric or electronic device according to claim 8, wherein a moisture content of the dopant solution is 1 wt.% or less.

10. An electric or electronic apparatus comprising the electric or electronic device described in claim 1 or 2.
